(19) Europäisches Patentamt
European Patent Office
Office européen des brevets

(11) Publication number: 0 321 563 B1

# (12) EUROPEAN PATENT SPECIFICATION

(45) Date of publication of patent specification: 06.11.91 Bulletin 91/45

(51) Int. Cl.$^5$: C07D 498/10, C07D 498/20, C09K 9/02, G03C 1/73

(21) Application number: 87904318.0

(22) Date of filing: 03.07.87

(86) International application number: PCT/JP87/00464

(87) International publication number: WO 88/00196 14.01.88 Gazette 88/02

## (54) SPIROOXAZINE COMPOUNDS, PHOTOSENSITIVE MATERIALS USING THEM, AND PROCESS FOR FORMING THEM.

(30) Priority: 08.07.86 JP 160124/86
20.10.86 JP 249250/86
20.10.86 JP 249251/86
01.06.87 JP 134954/87

(43) Date of publication of application: 28.06.89 Bulletin 89/26

(45) Publication of the grant of the patent: 06.11.91 Bulletin 91/45

(84) Designated Contracting States: DE GB

(56) References cited:
EP-A- 0 171 909
EP-A- 0 277 639
FR-A- 2 093 370
JP-A- 6 263 587

(73) Proprietor: NISSAN MOTOR CO., LTD.
No.2, Takara-cho, Kanagawa-ku
Yokohama-shi Kanagawa-ken 221 (JP)

Proprietor: MITSUBISHI KASEI CORPORATION
5-2, Marunouchi 2-chome Chiyoda-ku
Tokyo 100 (JP)

(72) Inventor: TATEOKA, Yasuo
2-18-6-303, Ikego
Zushi-shi Kanagawa 249 (JP)
Inventor: ITO, Masashi
3-68, Oppama Higashicho
Yokosuka-shi Kanagawa 237 (JP)
Inventor: MAEDA, Shuichi
3-29-16, Musashidai Hidakamachi
Iruma-gun Saitama 350-12 (JP)
Inventor: KIMURA, Michiyo
Green-Heights Ayabe 203 824-1, Imaizumi
Hadano-shi Kanagawa 257 (JP)
Inventor: MITSUHASHI, Kazuo
Popuragaoka Coop 9-106 2-11-3, Naruse
Machida-shi Tokyo 194 (JP)

(74) Representative: Vossius & Partner
Siebertstrasse 4 P.O. Box 86 07 67
W-8000 München 86 (DE)

EP 0 321 563 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel spiro-oxazine compounds having photochromic properties; photosensitive materials comprising a base having provided thereon a photosensitive layer containing a resin having dissolved or dispersed therein a spiro-oxazine compound; and to a process for producing photosensitive materials by film forming or molding a resin containing a spiro-oxazine compound.

It is hitherto known that spiro-oxazine compounds exhibit photochromism, developing a color or fading a color on light irradiation, and various photochromic photosensitive materials utilizing these compounds have been proposed.

For example, Japanese Patent Publication No. 45-28892 discloses photochromic materials containing spiro-naphthoxazine compounds having the formula:

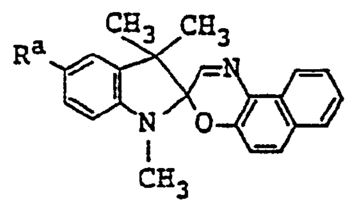

(wherein $R^a$ represents a hydrogen atom, a halogen atom, a cyano group, or an alkyl or alkoxy group having from 1 to 8 carbon atoms).

Japanese Patent Publication No. 49-48631 discloses a photochromic photosensitive material comprising a high polymeric substance having dispersed therein spiro-naphthoxazine compounds having the formula:

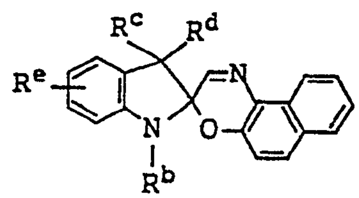

(wherein $R^b$ represents $-(CH_2)_nCOOH$, $-(CH_2)_nCN$ or $-(CH_2)_nCOOR$, (wherein R represents an alkyl group having from 1 to 5 carbon atoms); $R^c$ and $R^d$ each represents an alkyl group having from 1 to 5 carbon atoms; $R^e$ represents a hydrogen atom, an alkyl group having from 1 to 5 carbon atoms, a nitro group, a cyano group, an alkoxy carbonyl group having from 2 to 6 carbon atoms, or an alkoxy group having from 1 to 5 carbon atoms).

JP-A-55-36284 (the term "JP-A" as used herein means an "unexamined published Japanese patent application") discloses photochromic compounds having the formula:

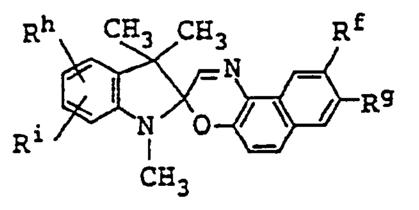

(wherein one of $R^f$ and $R^g$ represents a halogen atom or a lower alkoxy group with the other representing a hydrogen atom; and $R^h$ and $R^i$ each represents a hydrogen atom, a lower alkyl group, a lower alkoxy group, or a halogen atom).

U.S. Patent 4,342,668 proposes photochromic compounds having the formula:

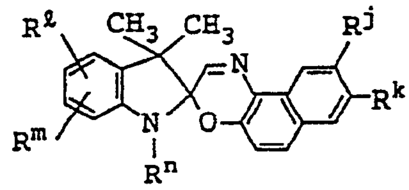

(wherein one of $R^j$ and $R^k$ represents a halogen atom or a lower alkoxy group, with the other representing a hydrogen atom; $R^\ell$ and $R^m$ each represents a hydrogen atom, a lower alkyl group, a lower alkoxy group, or a halogen atom; and $R^n$ represents an alkyl group having from 2 to 10 carbon atoms).

Tetrahedron Letters, Vol. 22, No. 40, 3945 proposes photochromic compounds having the formula:

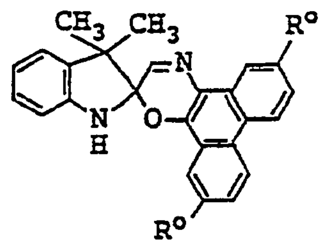

$R^o$=H, $NO_2$

JP-A-60-112880 proposes spiro-pyridobenzoxazine compounds having the formula shown below and a photochromic photosensitive material comprising a high polymeric substance having dispersed therein such compounds having the formula:

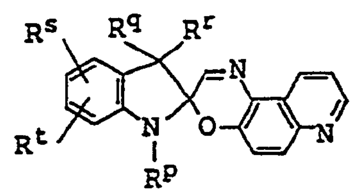

[wherein $R^p$ represents an alkyl group having from 1 to 8 carbon atoms, a phenyl group, a phenalkyl group having from 1 to 4 carbon atoms, an alkyl group, or a mono- or di-subsituted phenyl group where the substituent is selected from alkyl groups having from 1 to 4 carbon atoms or alkoxy groups having from 1 to 5 carbon atoms; $R^q$ and $R^r$ each represents an alkyl group having from 1 to 5 carbon atoms, a phenyl group, an alkoxy group having from 1 to 5 carbon atoms, a mono- or di-substituted phenyl group, or a benzyl group, or $R^q$ and $R^r$ are taken together to form an alicyclic ring, a norbornyl ring or an adamantyl ring containing from 6 to 8 carbon atoms (inclusive of a spiro carbon atom); and $R^s$ and $R^t$ each represents a hydrogen atom, an alkyl group having from 1 to 5 carbon atoms, a halogen atom, an alkoxy group having from 1 to 5 carbon atoms, a nitro group, a cyano group, or an alkoxycarbonyl group having from 1 to 8 carbon atoms].

Photochromic spiro-pyrans and their use for the preparation of direct positive photosensitive layers are also disclosed in FR-A-2 093 370.

However, these conventional spiro-oxazine compounds were not satisfactory in density and fastness of a developed color.

On the other hand, photochromic photosensitive materials are generally used in the form of a film comprising a high-molecular compound having dissolved or dispersed therein the spiro-oxazine compounds as exemplified by the above prior art.

A typical conventional process for film formation comprises once dissolving a photochromic compound in an appropriate solvent, mixing the solution with a separately prepared solution of a high-molecular matrix and a plasticizer, applying the resulting composition on a support, and removing the solvent by drying. For example, 0.5 g of a spiro-oxazine derivative of the formula:

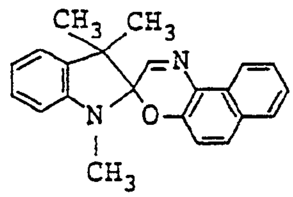

as a photochromic compound (hereinafter referred to as SP-1) is dissolved in 30 g of isopropanol (IPA), and the solution is mixed with a previously prepared solution of a high-molecular matrix such as 4.5 g of polyvinyl butyral (PVB) and 1.5 g of triethylene glycol di-2-ethylbutyrate (3GH) in 100 g of IPA, followed by thoroughly stirring. The resulting solution C is applied on a base (a glass is used here) by casting, spin coating, dip coating, spray coating or the like technique, followed by drying at 90°C for 30 minutes to remove the solvent thereby forming a film.

The high-molecular matrix includes not only thermoplastic resins but thermosetting resins. For pratical use, the thus obtained photochromic film on the glass base is laminated with other materials as described in JP-A-60-205429. For example, a PVB intermediate film (e.g., 760 µm thick) is laid on the photochromic layer on the glass base, and a glass plate is further laminate thereon. The material is set in a vacuum bag and maintained in vacuo under atmospheric pressure at 90°C for 30 minutes to effect preliminary press bonding. The material is then maintained in an autoclave at 140°C for 45 minutes under a pressure of 12.5 kg/cm$^2$ to complete press bonding.

The conventional spiro-oxazine compounds are poor in heat resistance as having a low melting point and a low heat decomposition temperature as shown in Table 1 below. It is unreasonable, therefore, to use these compounds in a film formation technique or a molding technique which comprises directly incorporating the compounds into a high-molecular matrix and molding the resin compounds into a film or other molded articles. When the compounds are applied to this molding technique, productivity attained is very low.

TABLE 1

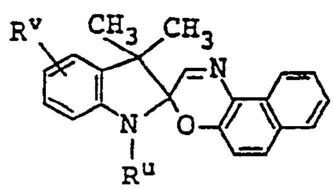

| Substituent $R^u$ | Substituent $R^v$ | Melting Point (°C) | Heat Decomposition Temperature (°C) |
|---|---|---|---|
| $-CH_3$ | - | 107 | 215 |
| $-CH_3$ | $-Cl$ | 111 | 218 |
| $-C_{18}H_{37}$ | - | 117 | 208 |
| $-C_2H_4OCH_3$ | - | 135 | 226 |
| $-C_3H_7(i)$ | - | 119 | 220 |
| $-C_2H_4OCH_3$ | $-Cl$ | 138 | 228 |
| $-C_3H_7(i)$ | $-Cl$ | 120 | 223 |

One object of this invention is to provide photochromic spiro-oxazine compounds having satisfactory heat resistance, developing a color having a high density and excellent fastness and having a stability of developing a color or fading a color repeatedly.

Another object of this invention is to provide a photosensitive material comprising a base having provided

thereon a photosensitive layer containing such photochromic compounds.

A further object of this invention is to provide a highly productive process for film formation or molding comprising directly incorporating such a photochromic compound into a high-molecular matrix, followed by film formation or molding.

That is, the gist of the present invention relates to spiro-oxazine compounds represented by formula (I):

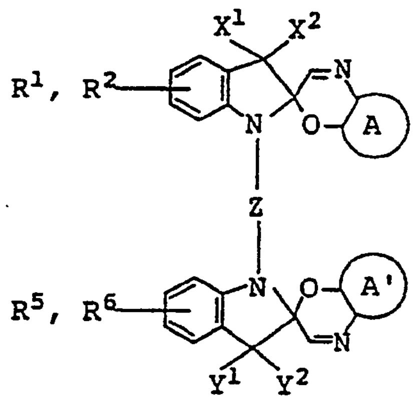

(I)

[wherein A and A', which are the same, represent

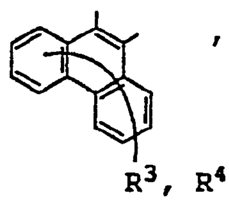

,

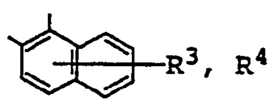

,

or

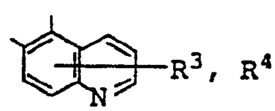

;

$X^1$, $X^2$, $Y^1$, and $Y^2$ each represents an alkyl group, or $X^1$ and $X^2$, or $Y^1$ and $Y^2$ are linked to each other to form a cyclohexane ring; Z represents a straight chain or branched chain alkylene group which may contain therein

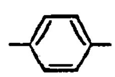 , 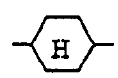 , 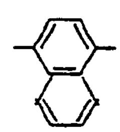

or

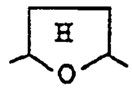

(each of which may have a substituent), or an oxygen atom; or an arylene group which may be substituted and $R^1$, $R^2$, $R^3$, $R^4$, $R^5$, and $R^6$ each represents a hydrogen atom, a halogen atom, an alkyl group, an alkoxy group, an alkoxycarbonyl group, an alkoxysulfonyl group or an alkylsulfonyl group] and photosensitive materials using the compounds and a process for forming the materials.

Figures 1 and 2 each shows an absorption spectrum of two embodiments (Examples 5 and 9) of the spiro-oxazine compounds according to the present invention in the state where a color is developed by ultraviolet irradiation. An ordinate shows an absorbance and an abscissa shows a wavelength.

Figure 3 is a graph showing a relation between films using SP-2 and SP-1 as a comparison, according to Example 13, in terms of a relation between time (ordinate, second) irradiating light emitted from a xenon lamp on the film and change in optical density (abscissa, ΔOD (at 610 nm)).

And Figure 4 is a graph showing the same relation as shown in Figure 3 between films using SP-3 and SP-4 as a comparison, according to Example 14. In the Figure an ordinate shows the same as of Figure 3, and a unit shown in an abscissa shows in minute.

Further, in both Figures 3 and 4, A is in a light-on state, while B is in a light-off state.

A constitution of the spiro-oxazine compounds represented by formula (I) according to the present invention is described in detail below.

In formula (I), $X^1$, $X^2$, $Y^1$, and $Y^2$ each represents an alkyl group containing from 1 to 23 carbon atoms, preferably from 1 to 4 carbon atoms, or $X^1$ and $X^2$, or $Y^1$ and $Y^2$ may be linked to each other to form a cyclohexyl ring. $X^1$, $X^2$, $Y^1$, and $Y^2$ each preferably represents a methyl, ethyl, propyl or butyl group, with a methyl group being more preferred, or a cyclohexane ring formed by linking $X^1$ and $X^2$, or $Y^1$ and $Y^2$.

Z represents a straight chain or branched chain alkylene group or a substituted or unsubstituted arylene group. The alkylene group may contain therein an oxygen atom or a group selected from

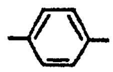 , 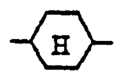 , 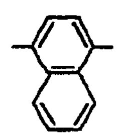

and

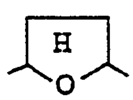

(each of which may be substituted). Such an alkylene group includes $-(CH_2)_n-$, $-CH_2-Q-CH_2-$, and

$$-[(CH_2)_mO]_\ell-(CH_2)_m-,$$

wherein Q represents

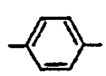 , 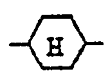 ,

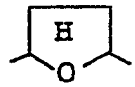

or

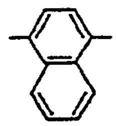 ;

n represents an integer of from 1 to 10; and ℓ and m each represents 1 or 2, with $-(CH_2)_n-$, wherein n is from 4 to 8 being preferred. Specific examples of the alkylene group are $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_{10}-$,

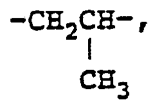

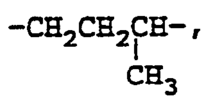 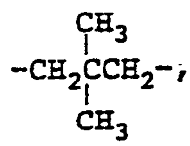

$-C_2H_4OC_2H_4-$, $-(CH_2)_2O(CH_2)_2O(CH_2)_2-$,

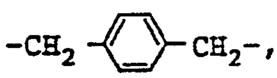 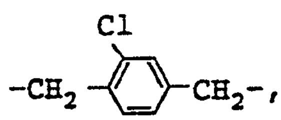 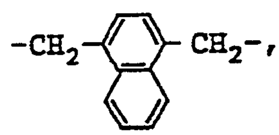

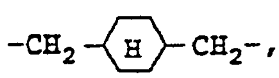

and

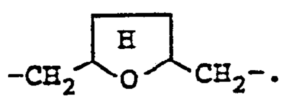

Z represents arylene group which may be substituted, includes

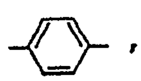

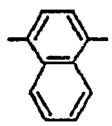 , 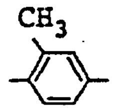 , 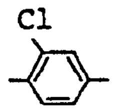

and

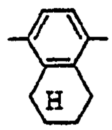

A and A', which are the same, represent

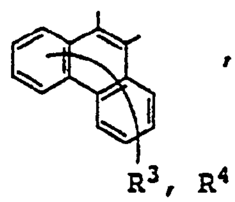 ,

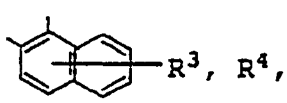

or

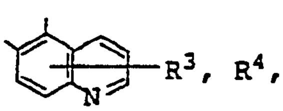

with

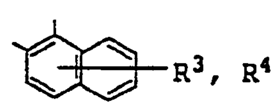

being preferred.

$R^1$, $R^2$, $R^3$, $R^4$, $R^5$, and $R^6$ each represents a hydrogen atom; a halogen atom, e.g., chlorine, bromine iodine; an alkyl group, for example, having from 1 to 6 carbon atoms; an alkoxy group, for example, having from 1 to 6 carbon atoms; an alkoxycarbonyl group, for example, having from 1 to 6 carbon atoms; an alkoxysulfonyl group, for example, having from 1 to 6 carbon atoms, e.g., methoxysulfonyl and ethoxysulfonyl; or an alkylsulfonyl group having from 1 to 6 carbon atoms, e.g., methylsulfonyl and ethylsulfonyl.

The preferred compounds are those wherein $R^1$, $R^2$, $R^5$, and $R^6$ each represents a hydrogen atom, a halogen atom, an alkyl group having from 1 to 6 carbon atoms, an alkoxycarbonyl group having from 1 to 6 carbon atoms, an alkylsulfonyl group having from 1 to 6 carbon atoms, or an alkoxysulfonyl group having from 1 to 6 carbon atoms; and $R^3$ and $R^4$ each represents a hydrogen atom, an alkoxy group having from 1 to 6 carbon atoms, a halogen atom, or an alkyl group having from 1 to 6 carbon atoms.

More preferred are those compounds wherein $R^1$, $R^2$, $R^5$, and $R^6$ each represents a hydrogen atom, an alkyl group having from 1 to 4 carbon atoms, an alkoxycarbonyl group having from 1 to 4 carbon atoms, an

alkoxysulfonyl group having from 1 to 4 carbon atoms, or an alkoxysulfonyl group having from 1 to 4 carbon atoms; and $R^3$ and $R^4$ each represents a hydrogen atom, an alkoxy group having from 1 to 4 carbon atoms, a halogen atom, or an alkyl group having from 1 to 4 carbon atoms.

Still more preferred are those compounds wherein $R^1$, $R^2$, $R^5$, and $R^6$ each represents a hydrogen atom, an alkyl group having 1 or 2 carbon atoms, an alkoxycarbonyl group having 1 or 2 carbon atoms, an alkylsulfonyl group having 1 or 2 carbon atoms, or an alkoxysulfonyl group having 1 or 2 carbon atoms; $R^3$ and $R^4$ each represents a hydrogen atom, an alkoxy group having 1 or 2 carbon atoms, a halogen atom, or an alkyl group having 1 or 2 carbon atoms; $X^1$, $X^2$, $Y^1$ and $Y^2$ each represents a methyl group; Z represents $-(CH_2)_n-$, wherein n is from 4 to 8; and A and A′ both represent

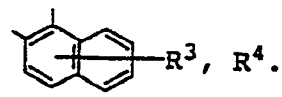

Still more preferred are those compounds of the formula I wherein A and A′, which are the same, represent

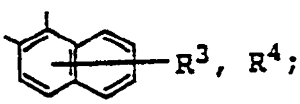

$R^1$, $R^2$, $R^5$, and $R^6$ each represents a hydrogen atom or a methyl group; and $R^3$ and $R^4$ each represents a hydrogen atom or a methoxy group.

The compounds of the present invention can be prepared, for example, by reacting a 2,3,3-trimethylindolenine derivative represented by formulae (II) and (III):

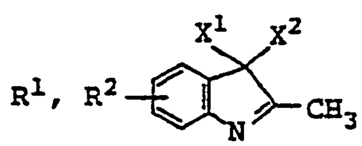

(II)

(wherein $X^1$, $X^2$, $R^1$ and $R^2$ are the same as defined above),

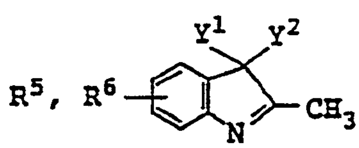

(III)

(wherein $Y^1$, $Y^2$, $R^5$, and $R^6$ are the same as defined above), with a dihalide compound represented by formula (IV):

M-Z-M (IV)

(wherein M represents a halogen atom; and Z is as defined above), and then reacting the resulting compound with a 1-nitroso-2-naphthol derivative represented by formula (V):

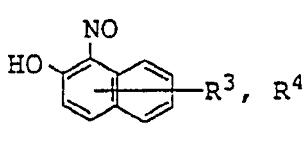

(V)

a 9-nitroso-10-hydroxyphenalene derivative represented by formula (VI):

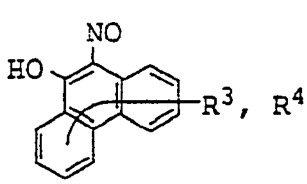

(VI)

or a 5-nitroso-6-hydroxyquinoline derivative represented by formula (VII):

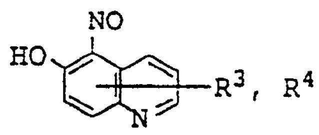

(VII)

(wherein $R^3$ and $R^4$ are the same as defined above).

The alkylation reaction using the dihalide can be carried out in the absence of a solvent or in the presence of a non-polar solvent such as aromatic solvents e.g., chlorobenzene and dichlorobenzene, at a temperature of from 80 to 200°C. The reaction is preferably carried out in the absence of a solvent at a temperature between 130°C and 150°C.

The reaction with a 1-nitroso-2-naphtol derivative of the formula (V), a 9-nitroso-10-hydroxyphenalene derivative of the formula (VI), or a 5-nitroso-6-hydroxyquinoline derivative of the formula (VII), is carried out in a polar or non-polar solvent, such as alcoholic solvents, e.g., methanol, ethanol, propanol and butanol, ketone solvents, e.g., acetone and methyl ethyl ketone, and halogenated hydrocarbon solvents, e.g., dichloromethane and trichloroethane, in the presence of basic catalysts, e.g., piperidine, piperazine and morpholine, at a temperature ranging from 0 to 200°C. The reaction is preferably effected in ethanol, methyl ethyl ketone or acetone in the presence of a piperidine catalyst at 40 to 120°C.

The compounds of the present invention can also be prepared by reacting a compound represented by formula (VIII):

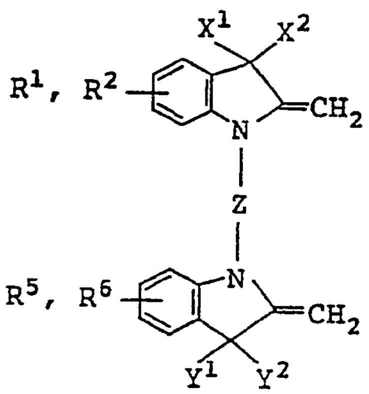

(VIII)

(wherein $X^1$, $X^2$, $Y^1$, $Y^2$, Z, $R^1$, $R^2$, $R^5$, and $R^6$ are as defined above), with a 1-nitroso-2-naphthol derivative of formula (V),

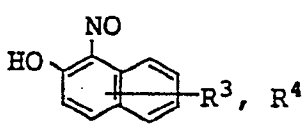

(V)

a 9-nitroso-10-hydroxyphenalene derivative of formula (VI)

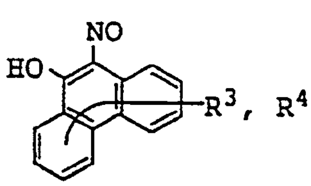

(VI)

or a 5-nitroso-6-hydroxyquinoline derivative of formula (VII).

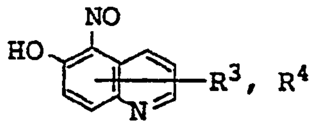

(VII)

(wherein $R^3$ and $R^4$ are the same as defined above).

This reaction is usually carried out in a polar or non-polar solvent, such as alcoholic solvents e.g., methanol, ethanol, propanol, and butanol, ketone solvents e.g., acetone and methyl ethyl ketone and halogenated hydrocarbon solvents e.g., dichloromethane and trichloroethane, at 0 to 200°C, preferably 40 to 120°C.

The compounds of the present invention are novel and exhibit photochromism. The compounds develop a color on ultraviolet irradiation and then return to their original colorless state on irradiation of visible light or heating to 80°C or higher. This color change is repeatable.

A further subject matter of the invention is a photosensitive material comprising a base having provided thereon a photosensitive layer containing a resin having dissolved or dispersed therein the spiro-oxazine compound of the present invention as mentioned above this material can be obtained in a usual manner.

The resin in which the compound is dissolved or dispersed includes polyester resins, polystyrene resins, polyvinyl butyral resins, polyvinylidene chloride, polyvinyl chloride, polymethyl methacrylate, polyvinyl acetate, cellulose acetate, epoxy resins, and phenolic resins. The solvent to be used includes carbon tetrachloride, benzene, cyclohexane, methyl ethyl ketone, tetrachloroethane, toluene, ethanol, and ethyl cellosolve.

The compounds of the present invention are usually used in an amount of from 3 to 50%, preferably from 5 to 20%, based on the resins.

For the purpose of improving stability or light-resistance, the photosensitive layer may contain additives, such as a transition metal chelate compound (e.g., acetylacetonato chelates, bisphenyldithiol, salicylaldehyde oxime, and bisdithiol-α-diketone) as a quencher for single state oxygen, an ultraviolet absorbent and an antioxidant.

The photosensitive layer can be formed by a commonly employed coating method, such as doctor blade coating, casting, spinner coating, dip coating, and the like. The thickness of the photosensitive layer is usually from 0.5 µm to 1.0 mm, preferably from 5 to 100 µm.

The base to be used in the present invention may be either transparent or opaque. In photosensitive materials having a base on both sides of the photosensitive layer, at least one of the two bases should be transparent. The base includes glass plates, plastic films, paper, metal plates or foils, and composite materials thereof. Preferred of them are glass plates and plastic films from various aspects. Implicit in the plastic are acrylic resins, methacrylic resins, vinyl acetate resins, vinyl chloride resins, polyethylene resins, polypropylene resins, polycarbonate resins, polypropylene resins, polycarbonate resins, polysulfone resins, etc.

Still a further subject matter of the invention is a molding process for producing a photosensitive material comprising incorporating a compound of formula (I) as mentioned above into a high-molecular material at a high temperature, followed by film formation or molding.

The high-molecular material to be used as a matrix includes all the resins capable of being injection molded, such as acrylic polymers, e.g., polymethyl methacrylate (PMMA), styrene polymers, e.g., polystyrene, polycarbonate, polyesters, polyethers, e.g., polyethylene oxide, polyamides, e.g., nylon 6, polyolefins, e.g., polyethylene, cellulose polymers e.g., ethyl cellulose, polyvinyl chloride, polyurethane, polivinyl alcohol, polyvinyl butyral, polyvinyl acetate, polyglycidyl methacrylate, poly-N-vinylcarbazole, polyethylene vinylacetate, polyvinylidene chloride, copolymers comprising monomers constituting these homopolymers, and mixtures thereof.

The amount of the photochromic compound of formula (I) to be incorporated into the high-molecular material usually ranges from 0.001 to 15% by weight, preferably from 0.01 to 5% by weight.

The molding can be performed by molding techniques requiring high temperatures, such as film extrusion, injection molding, vacuum forming, blow molding, press molding, injection molding of thermo-setting resins, pultrusion, RIM molding, RTM molding, and the like.

The present invention is now illustrated in greater detail by way of Examples, but it should be understood that the present invention is not deemed to be limited thereto.

EXAMPLE 1

A mixture of 15.9 g of 2,3,3-trimethylindolenine of formula:

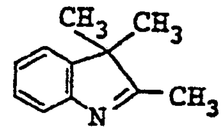

and 10.8 g of dibromobutane having a formula: ($BrC_4H_8Br$) was allowed to react at 110°C for 4 hours. After cooling to room temperature, 200 ml of ethanol was added thereto, and to the mixture were further added 17.3 g of 1-nitroso-2-naphthol of formula:

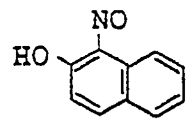

and 10.1 g of triethylamine. The mixture was allowed to react at reflux for 2 hours in a nitrogen atmosphere. After cooling, the reaction mixture was extracted with chloroform, and the extract was dried over sodium sulfate. The solvent was removed by distillation, and the residue was purified by column chromatography (benzene as an eluent) to obtain colorless crystals of spiro-oxazine compound of formula:

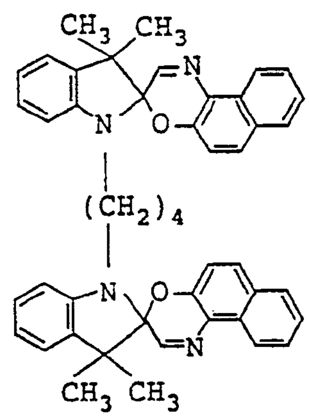

Melting point: 225-227°C

Mass spectrum (70 eV, 280°C): m/e 682 ($M^+$)
667 ($M^+$-15)
170 (base peak)

A solution consisting of 4 g of the compound, 10 g of a thermoplastic polyester resin ("Vylon 200" produced by Toyobo Co., Ltd.), and 100 g of methyl ethyl ketone was coated on a 100 μm thick polyester film (produced by DIAFOIL Co., Ltd.) by means of a bar coater No. 3, followed by drying at 75°C for 10 minutes.

The resulting photosensitive material was colorless in an ordinary state but turned to deep blue color ($\lambda_{max}$=606 nm) on irradiation of ultraviolet light. Then, it returned to its original colorless state on irradiation of visible light or heating at 80°C or more. These color changes could be effected repeatedly.

EXAMPLE 2

In the same manner as in Example 1, except for using 9.4 g of dibromoethane in place of the dibromobutane, a compound of formula shown below was obtained.

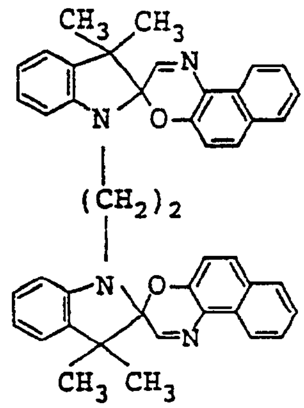

Mass Spectrum are as follows.

(Measurement Condition 70 eV, 270°C): m/e 654 ($M^+$)

639 ($M^+$-15)

170 (base peak)

A photosensitive material was produced by using the compound in the same manner as in Example 1. The sample developed a deep blue color ($\lambda_{max}$=606 nm) on ultraviolet irradiation and returned to its original colorless state on visible light irradiation or heating to 80°C or more. These color changes could be effected repeatedly.

EXAMPLE 3

In 1000 ml of methyl ethyl ketone was dissolved 42.0 g of a compound of formula:

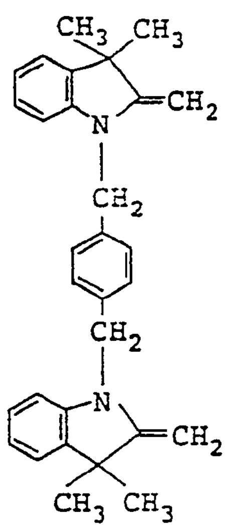

and to the solution was added 34.6 g of a compound of formula:

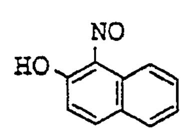

The mixture was refluxed for 3 hours and then cooled to room temperature. The solvent was removed by distillation, and the residue was separated and purified by column chromatography (benzene as an eluent) to obtain a spiro-oxazine compound of formula:

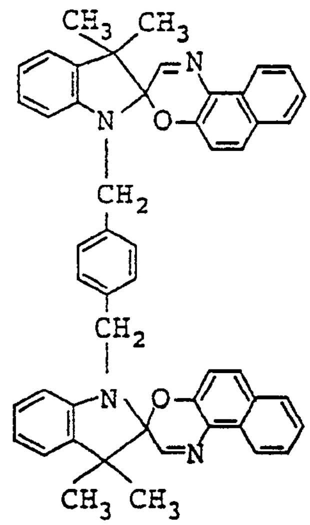

Mass Spectrum are as follows.

(Measurement Condition 70 eV, 300°C): m/e 730 ($M^+$)
715 ($M^+$-15)
170 (base peak)

A photosensitive material was produced by using the compound in the same manner as in Example 1. The resulting sample developed a deep blue color ($\lambda_{max}$=606 nm) on ultraviolet irradiation and then returned to its original colorless state on visible light irradiation or heating to 80°C or higher. These color changes could be effected repeatedly.

EXAMPLE 4

In the same manner as in Examples 1 to 3, spiro-oxazine compounds shown in Table 2 below were synthesized. The melting point and heat decomposition temperature of these compounds are also shown in Table 2.

A photosensitive material was produced by using each of these compounds in the same manner as in Example 1. Upon ultraviolet irradiation, the sample developed a color having tone and $\lambda_{max}$ as shown in Table 2 and, on visible light irradiation or heating to 80°C or higher it returned to its original colorless state. These color changes could be effected repeatedly.

TABLE 2

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-1 | 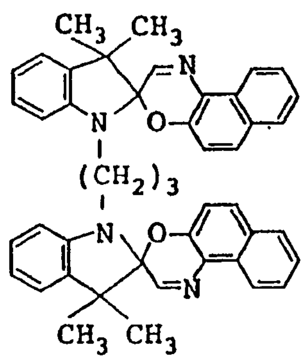 | Blue | 606 | 220 | 280 |
| 4-2 | 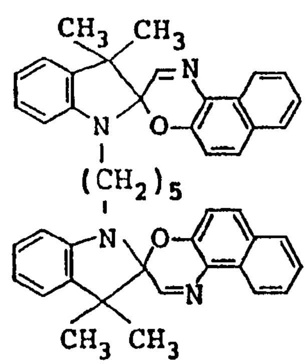 | Blue | 606 | | |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-3 | 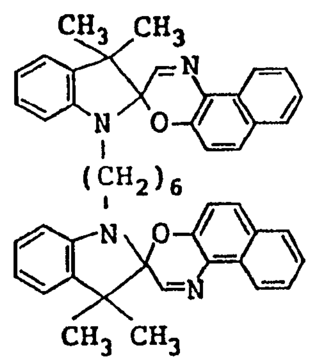 | Blue | 606 | 215 | 281 |
| 4-4 | 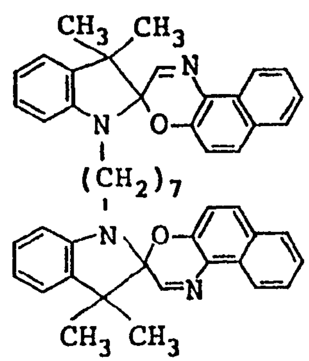 | Blue | 606 | 213 | 277 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-5 | 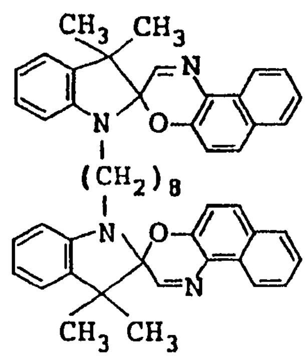 | Blue | 606 | 211 | 273 |
| 4-6 | 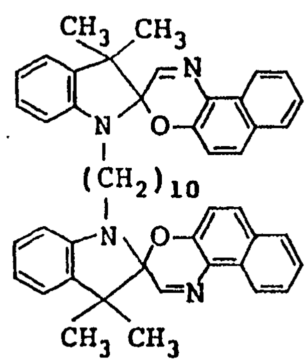 | Blue | 606 | 209 | 266 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-7 | 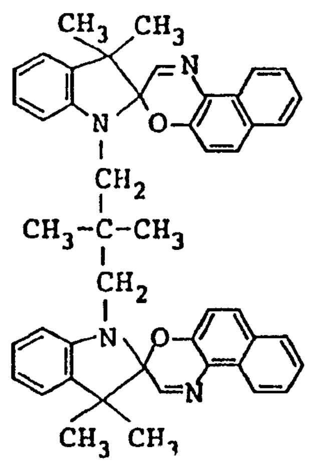 | Blue | 606 | 217 | 285 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-8 | 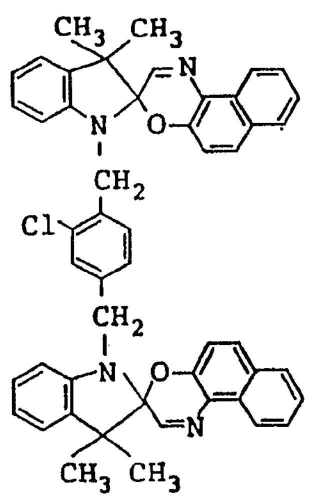 | Blue | 606 | 239 | 273 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-9 | 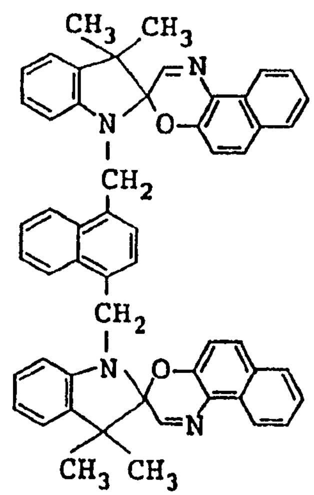 | Blue | 606 | 240 | 276 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-10 | 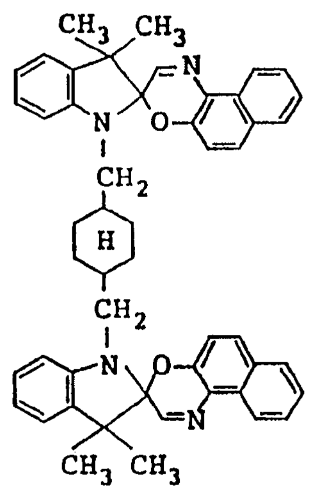 | Blue | 606 | 231 | 268 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-11 | 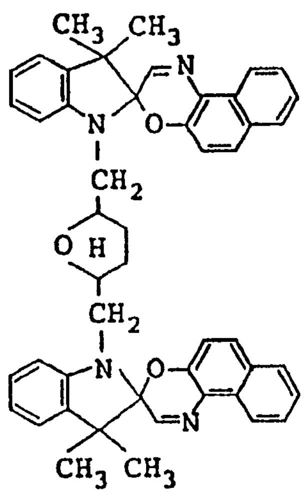 | Blue | 606 | 229 | 254 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-12 | 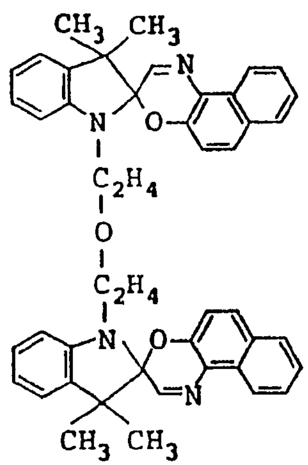 | Blue | 606 | 215 | 259 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-13 | 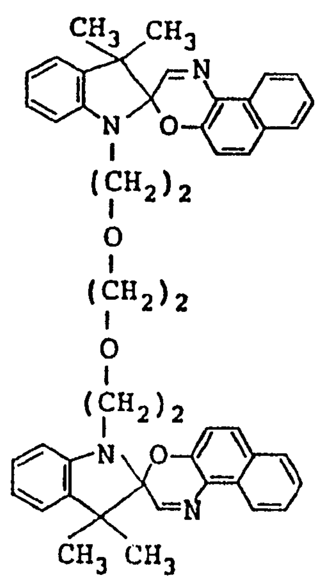 | Blue | 606 | 218 | 265 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-14 | 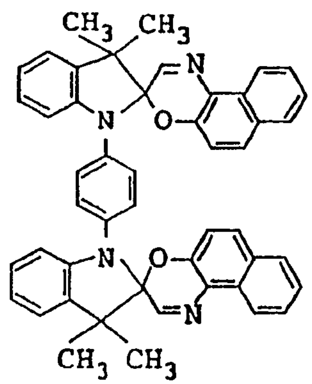 | Blue | 606 | 238 | 281 |
| 4-15 | 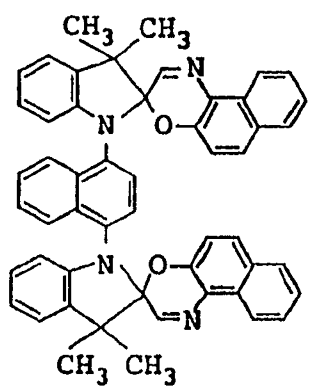 | Blue | 606 | 241 | 278 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-16 | 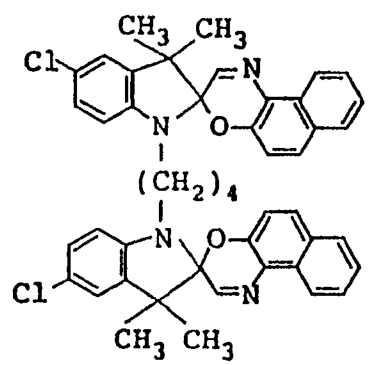 | Blue | 604 | 229 | 275 |
| 4-17 | 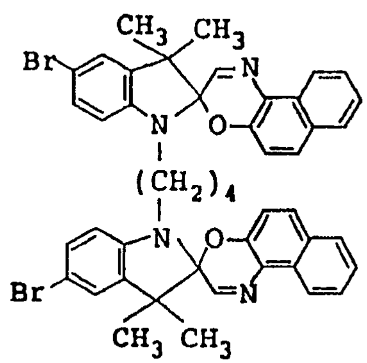 | Blue | 604 | 238 | 278 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-18 | 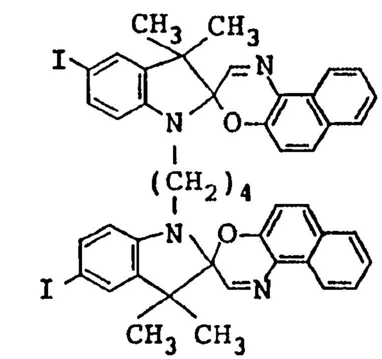 | Blue | 604 | 236 | 263 |
| 4-19 | 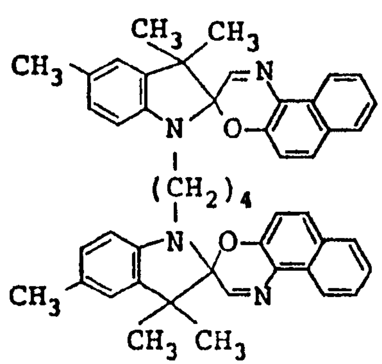 | Blue | 604 | 215 | 270 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-20 | 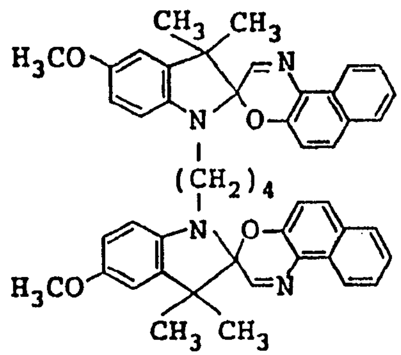 | Blue | 603 | 224 | 268 |
| 4-21 | 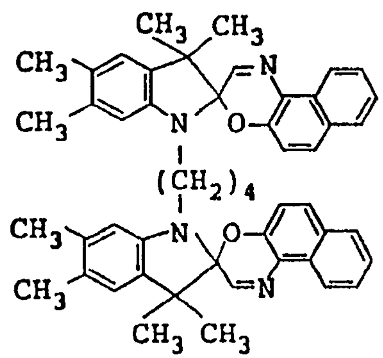 | Blue | 605 | 222 | 278 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-22 | 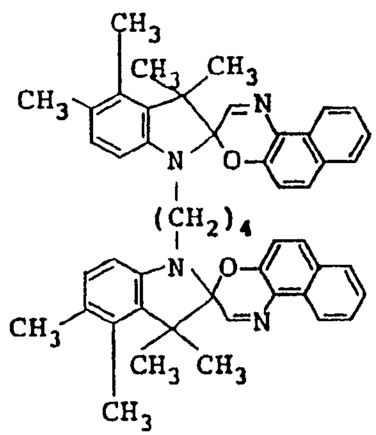 | Blue | 605 | 221 | 275 |
| 4-23 | 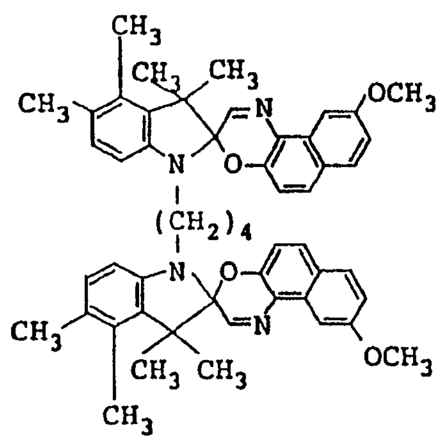 | Blue | 606 | 225 | 281 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-24 | 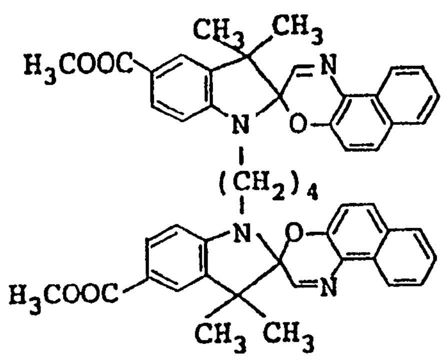 | Blue | 607 | 231 | 283 |
| 4-25 | 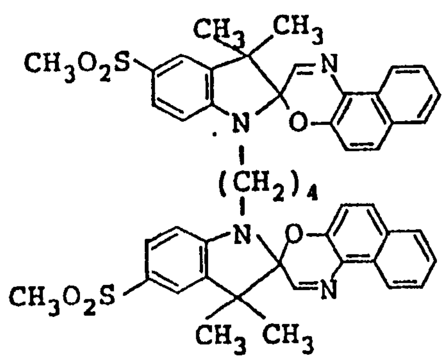 | Blue | 607 | 232 | 269 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-26 | 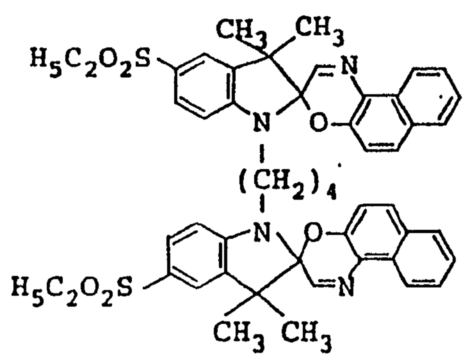 | Blue | 608 | 219 | 278 |
| 4-27 | 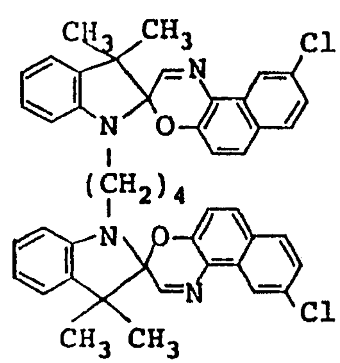 | Blue | 606 | 225 | 282 |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-28 | 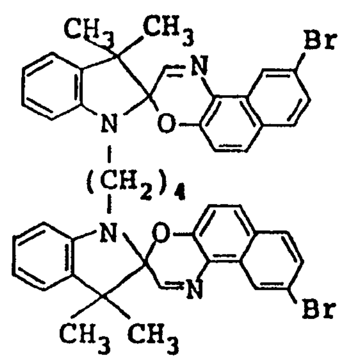 | Blue | 606 | 230 | 281 |
| 4-29 | 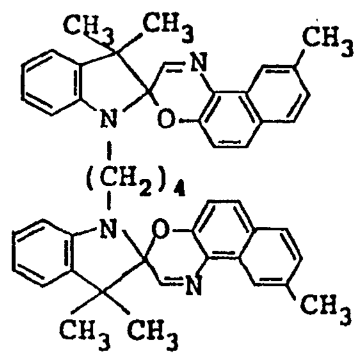 | Blue | 606 | 218 | 280 |

TABLE 2 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 4-30 | 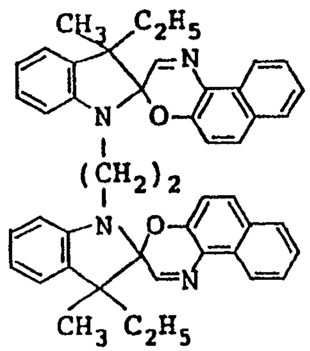 | Blue | 606 | | |
| 4-31 | 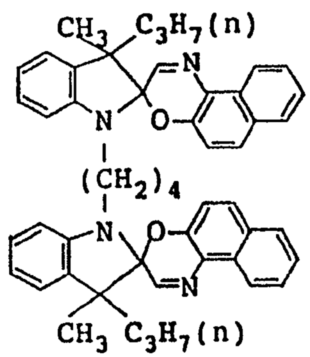 | Blue | 606 | | |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-32 | 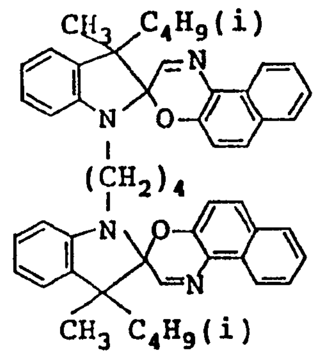 | Blue | 606 | | |
| 4-33 | 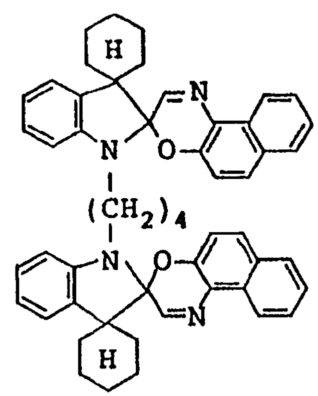 | Blue | 606 | | |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-34 | 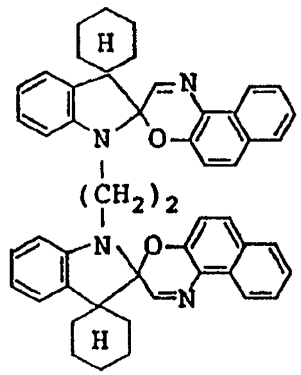 | Blue | 606 | | |
| 4-35 | 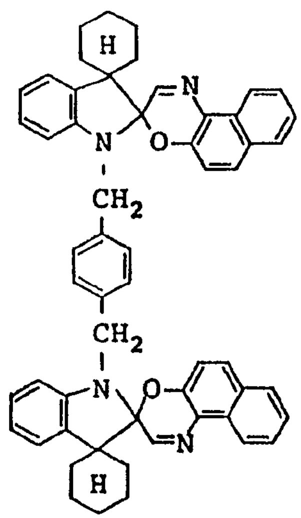 | Blue | 606 | | |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-36 | 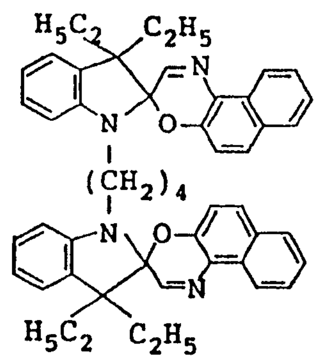 | Blue | 606 | | |
| 4-37 | 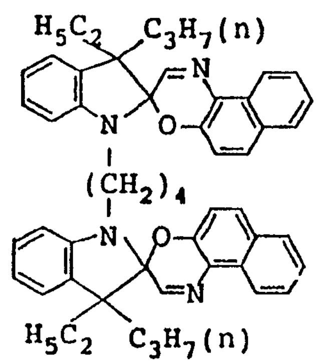 | Blue | 606 | | |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-38 | 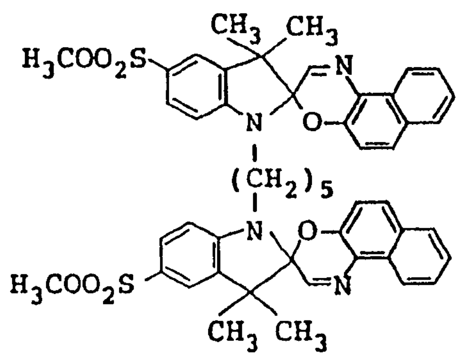 | Blue | 607 | | |
| 4-39 | 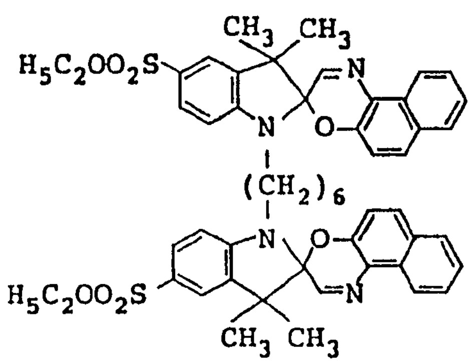 | Blue | 607 | | |

TABLE 2 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 4-40 | 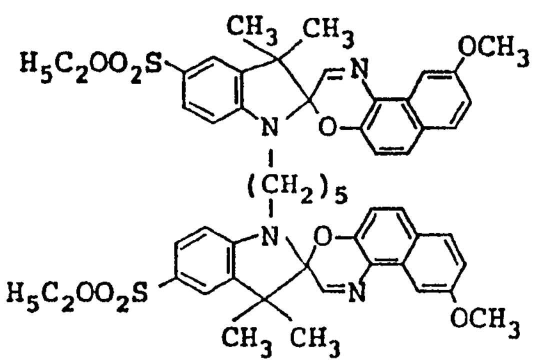 | Blue | 609 | | |

EXAMPLE 5

A mixture of 15.9 g of 2,3,3-trimethylindolenine of formula:

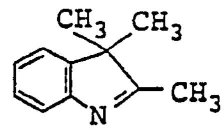

and 10.8 g of dibromobutane having a formula: ($BrC_4H_8Br$) was allowed to react at 110°C for 4 hours. After cooling to room temperature, 200 ml of ethanol was added thereto, and to the mixture was further added 22.3 g of 9-nitroso-10-hydroxyphenanthrene of formula:

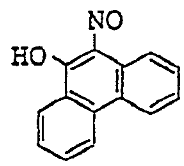

and 10.1 g of triethylamine. The mixture was allowed to react at reflux for 2 hours in a nitrogen atmosphere. After cooling, the reaction mixture was extracted with chloroform, and the extract was dried over sodium sulfate. The solvent was removed by distillation, and the residue was purified by column chromatography (benzene as an eluent) to obtain colorless crystals of spiro-oxazine compound of formula having a melting point of 237 to 238°C:

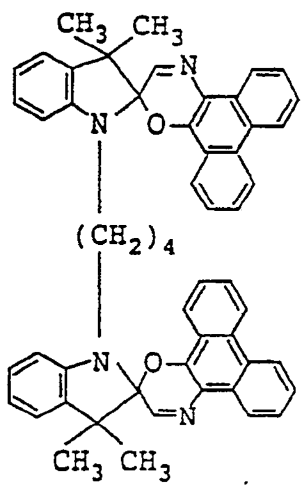

Mass Spectrum are as follows.

(Measurement Condition 70 eV, 300°C): m/e 782 ($M^+$)

767 ($M^+$-15)

219 (base peak)

A solution consisting of 4 g of the compound, 10 g of a thermoplastic polyester resin ("Vylon 200", produced by Toyobo Co., Ltd.), and 100 g of methyl ethyl ketone was coated on a polyester film (produced by DIAFOIL Co., Ltd. having 100 μm thick) by means of a bar coater No. 3, followed by drying at 75°C for 10 minutes.

The resulting photosensitive material was pale blue in an ordinary state but turned to deep blue ($\lambda_{max}$=600

nm) on irradiation of ultraviolet light. The absorption spectrum of the developed color is shown in Fig. 1. Then, it returned to its original state of pale blue on irradiation of visible light or heating at 50°C or more. These color changes could be effected repeatedly.

EXAMPLE 6

In the same manner as in Example 5, except for using 9.4 g of dibromoethane in place of the dibromobutane, a compound of formula shown below was obtained.

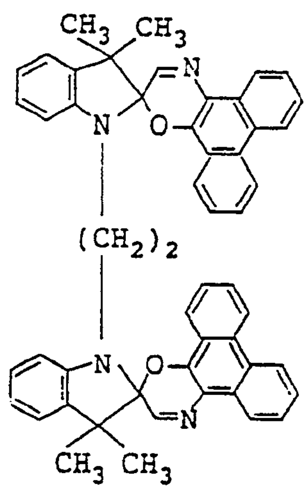

Mass Spectrum are as follows.

Measurement Conditions (70 eV, 280°C): m/e 754 ($M^+$)
739 ($M^+$-15)
219 (base peak)

A photosensitive material was produced by usingthe compound in the same manner as in Example 5. The sample developed a deep blue color ($\lambda_{max}$=600 nm) on ultraviolet irradiation and returned to its original pale blue state on visible light irradiation or heating to 50°C or higher. These color changes could be effected repeatedly.

EXAMPLE 7

In 1000 ml of methyl ethyl ketone was dissolved 42.0 g of a compound of formula:

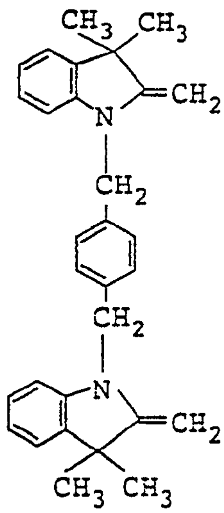

and to the solution was added 34.6 g of a compound of formula:

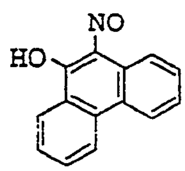

The mixture was refluxed for 3 hours and then cooled to room temperature. The solvent was removed by distillation, and the residue was purified by column chromatography (benzene as an eluent) to obtain a spiro-oxazine compound of formula:

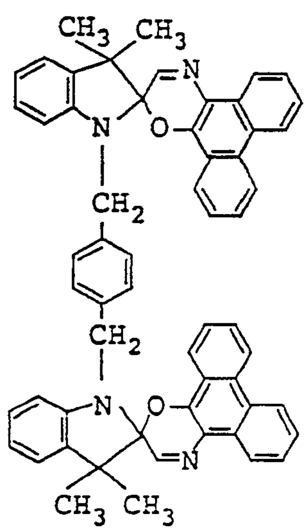

Mass Spectrum are as follows.

(Measurement Condition 70 eV, 320°C): m/e 830 ($M^+$)

815 ($M^+$-15)

219 (base peak)

A photosensitive material was produced by using the compound in the same manner as in Example 5. The resulting sample developed a deep blue color ($\lambda_{max}$=600 nm) on ultraviolet irradiation and then returned to its original state of pale blue on visible light irradiation or heating to 50°C or higher. These color changes could be effected repeatedly.

EXAMPLE 8

In the same manner as in Examples 5 to 7, spiro-oxazine compounds shown in Table 3 below were synthesized. The melting point and heat decomposition temperature of these compounds are also shown in Table 3.

A photosensitive material was produced by using each of these compounds in the same manner as in Example 5. Upon ultraviolet irradiation, the sample developed a color having tone and $\lambda_{max}$ as shown in Table 3 and, on visible light irradiation or heating to 80°C or higher it returned to its original pale blue state. These color changes could be effected repeatdely.

TABLE 3

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-1 | 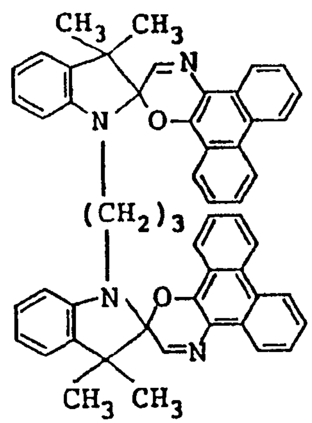 | Blue | 600 | | |
| 8-2 | 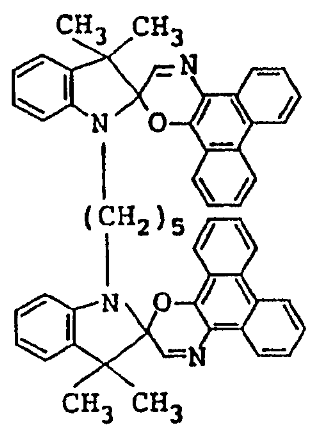 | Blue | 600 | | |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-3 | 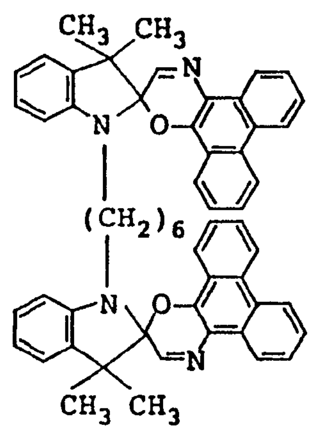 | Blue | 600 | 232 | 275 |
| 8-4 | 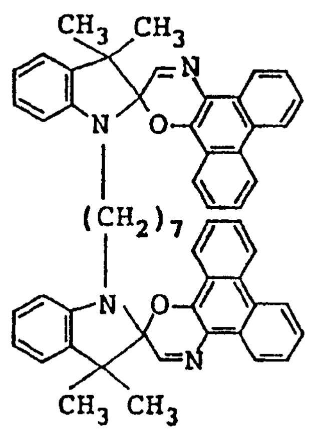 | Blue | 600 | 231 | 273 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-5 | 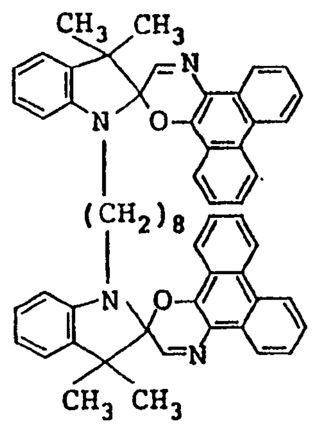 | Blue | 600 | 227 | 271 |
| 8-6 | 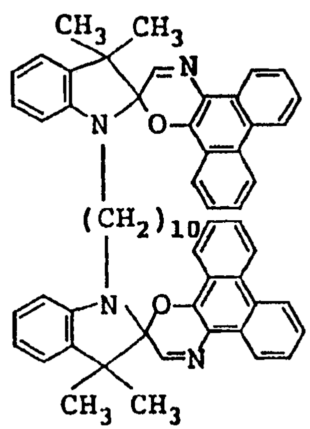 | Blue | 600 | 225 | 269 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-7 | 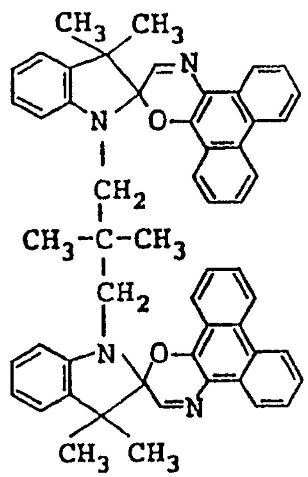 | Blue | 600 | 228 | 273 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-8 | 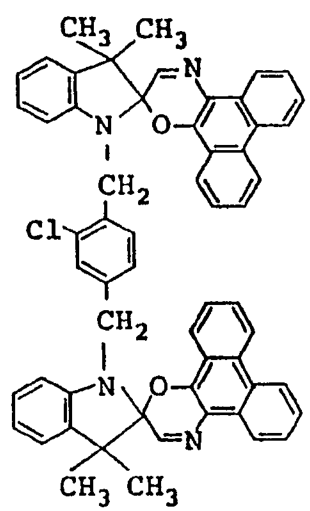 | Blue | 600 | 232 | 275 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-9 | 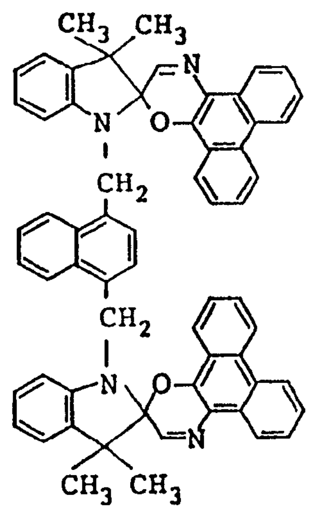 | Blue | 600 | 234 | 278 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-10 | 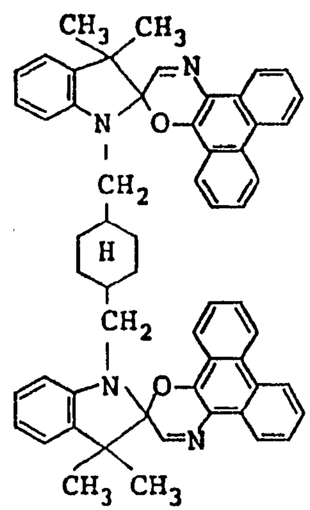 | Blue | 600 | 231 | 268 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-11 | 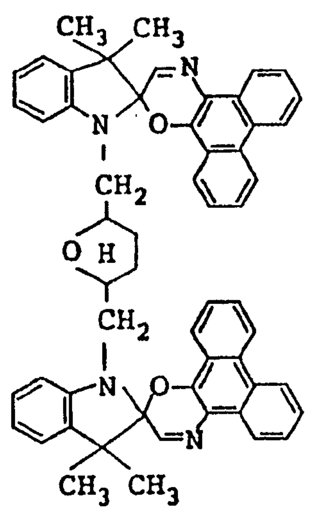 | Blue | 600 | 228 | 253 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-12 | 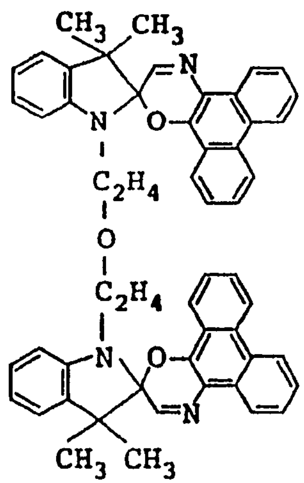 | Blue | 600 | 217 | 269 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-13 | 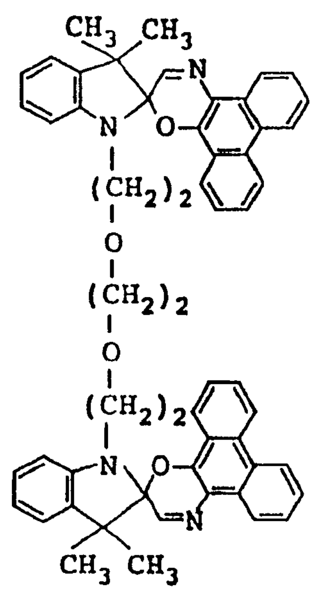 | Blue | 600 | 219 | 268 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-14 | 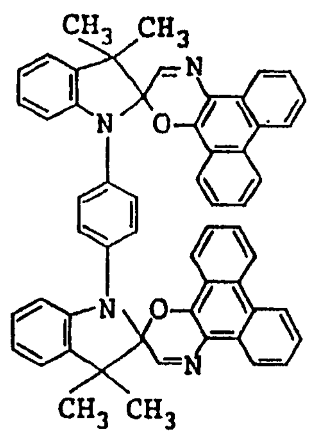 | Blue | 600 | 227 | 271 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-15 | 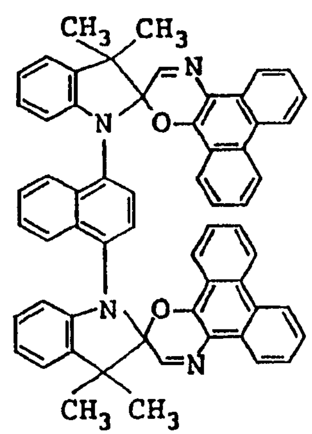 | Blue | 600 | 238 | 279 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
| --- | --- | --- | --- | --- | --- |
| 8-16 | 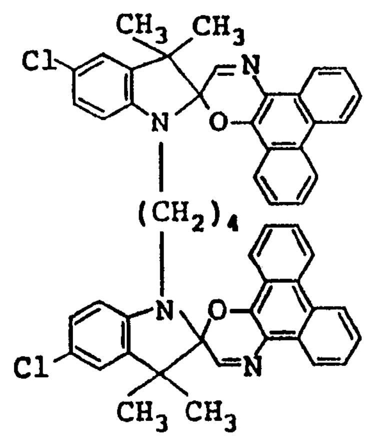 | Blue | 605 | 229 | 275 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-17 | 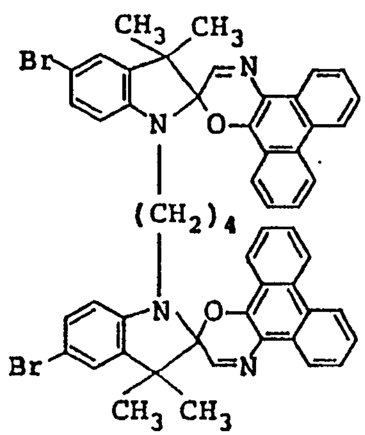 | Blue | 605 | 231 | 273 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
| --- | --- | --- | --- | --- | --- |
| 8-18 | 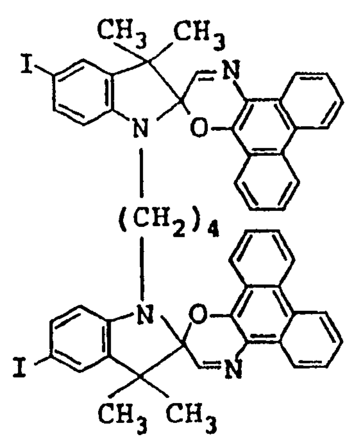 | Blue | 605 | 230 | 269 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-19 | 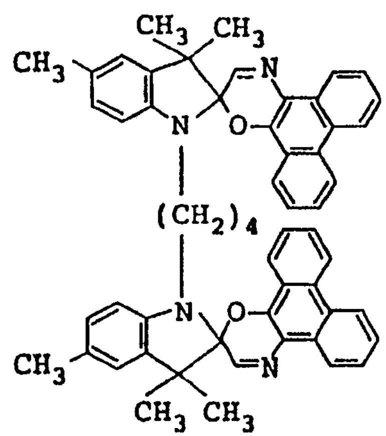 | Blue | 600 | 218 | 265 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-20 | 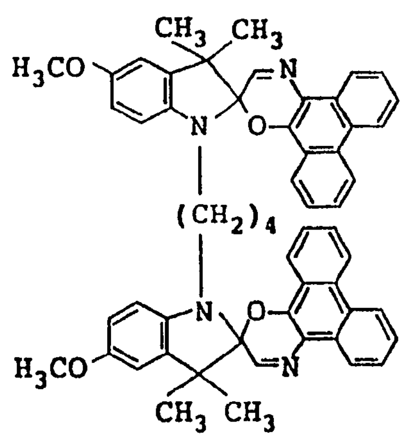 | Blue | 600 | 219 | 263 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-21 | 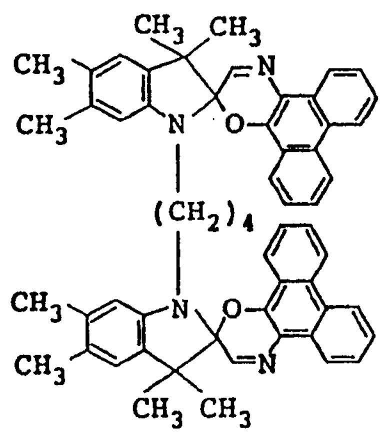 | Blue | 605 | 219 | 268 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-22 | 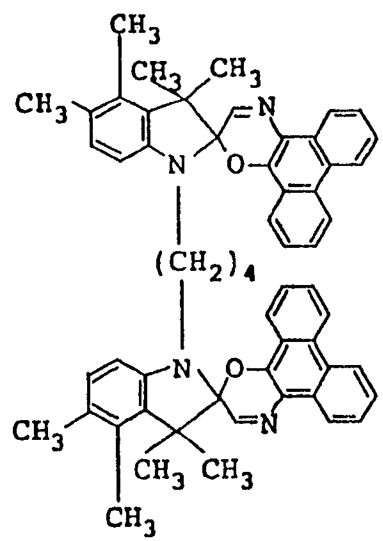 | Blue | 605 | 221 | 269 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-23 | 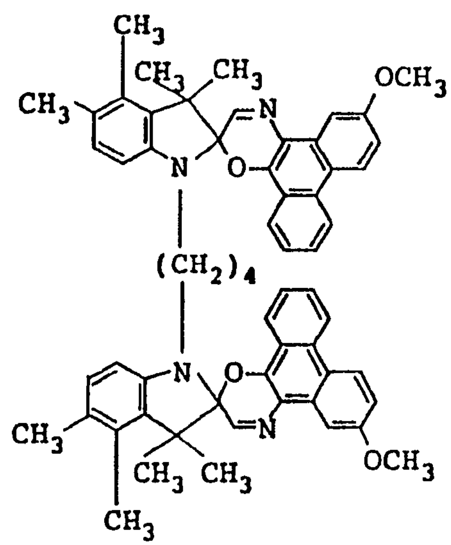 | Blue | 610 | 224 | 269 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-24 | 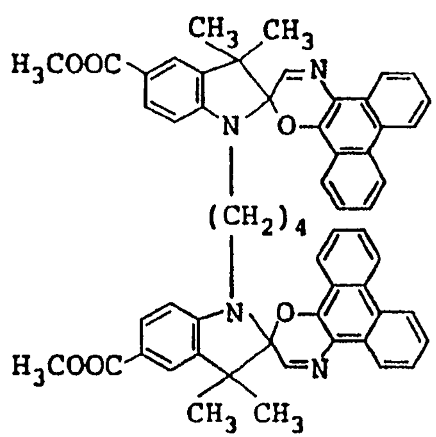 | Blue | 605 | 225 | 266 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-25 | 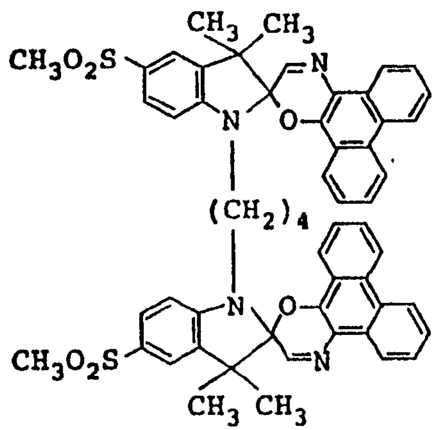 | Blue | 600 | 228 | 264 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-26 | 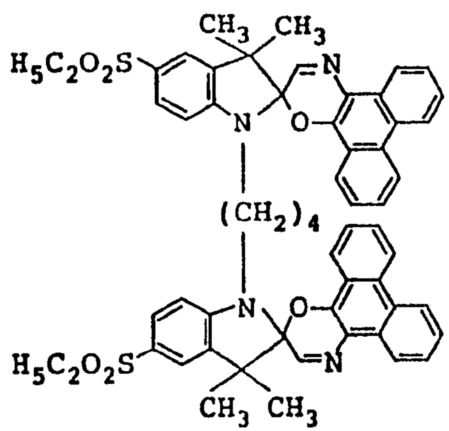 | Blue | 605 | 229 | 263 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-27 | 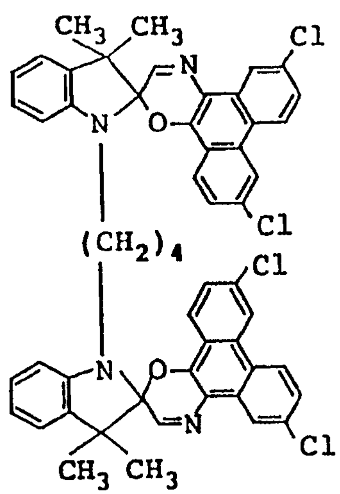 | Blue | 607 | 230 | 268 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-28 | 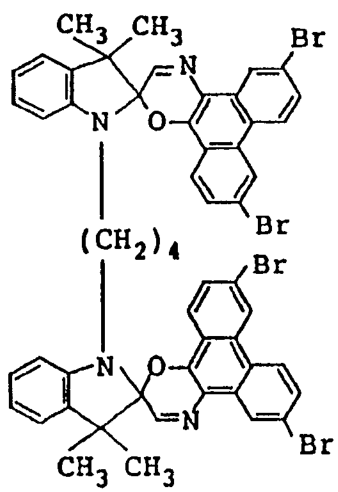 | Blue | 607 | 233 | 265 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-29 | 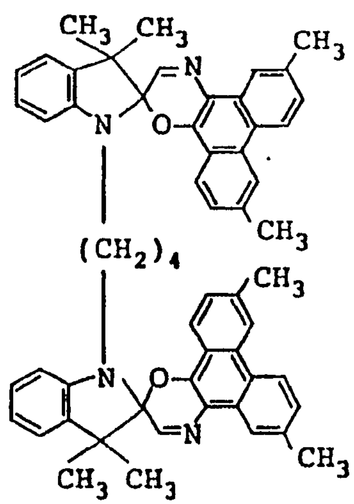 | Blue | 605 | 226 | 259 |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-30 | 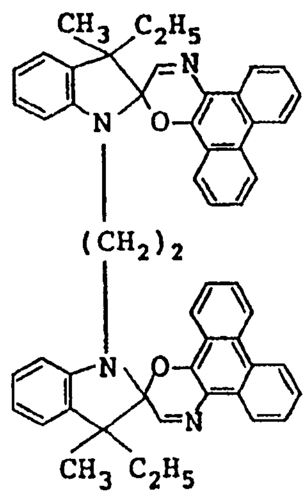 | Blue | 600 | 222 | 267 |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-31 | 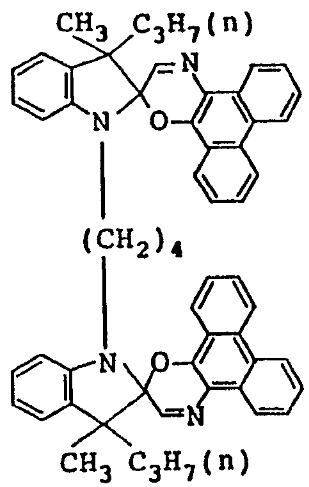 | Blue | 600 | | |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-32 | 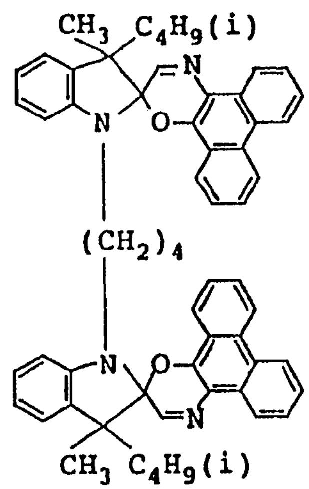 | Blue | 600 | | |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-33 | 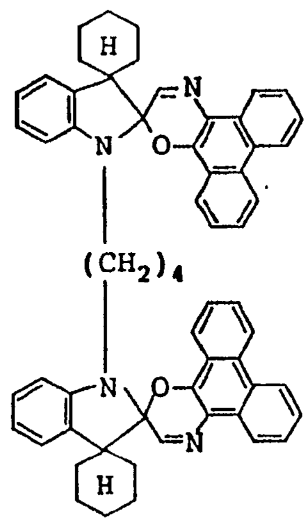 | Blue | 600 | | |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-34 | 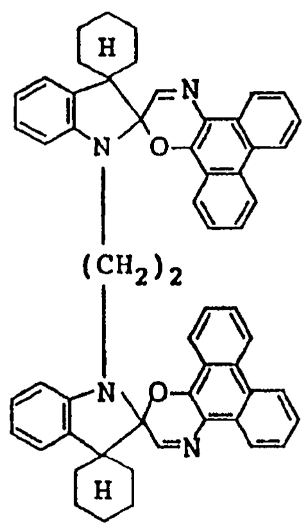 | Blue | 600 | | |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-35 | 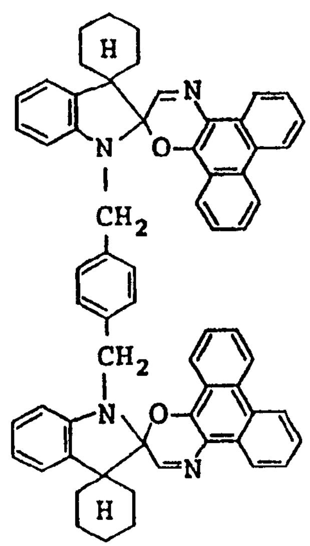 | Blue | 600 | | |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-36 | 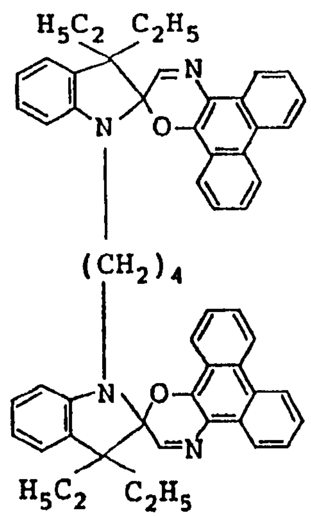 | Blue | 600 | | |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-37 | 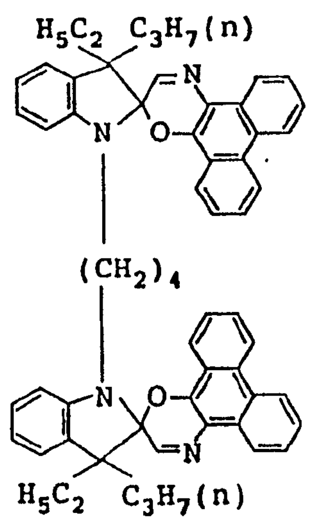 | Blue | 600 | | |

TABLE 3 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 8-38 | 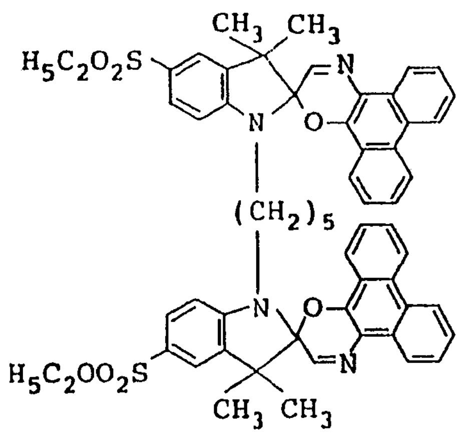 | Blue | 605 | | |

TABLE 3 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 8-39 | 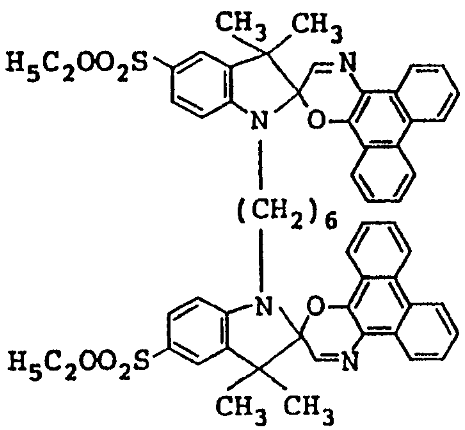 | Blue | 605 | | |

EXAMPLE 9

A mixture of 15.9 g of 2,3,3-trimethylindolenine of formula:

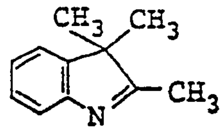

and 10.8 g of dibromobutane having a formula: ($BrC_4H_8Br$) was allowed to react at 110°C for 4 hours. After cooling to room temperature, 200 ml of ethanol was added thereto, and to the mixture were further added 17.3 g of 5-nitroso-6-hydroxyquinoline of formula:

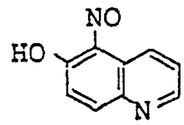

and 10.1 g of triethylamine. The mixture was allowed to react at reflux for 2 hours in a nitrogen atmosphere. After cooling, the reaction mixture was extracted with chloroform, and the extract was dried over sodium sulfate. The solvent was removed by distillation, and the residue was purified by column chromatography (benzene as an eluent) to obtain colorless crystals of spiro-oxazine compound of formula having a melting point of 210 to 214°C:

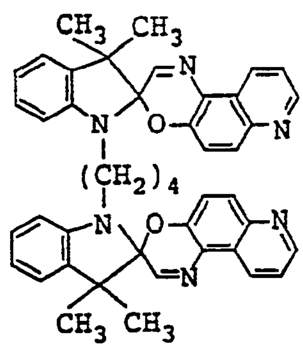

Mass Spectrum are as follows.

(Measurement Condition 70 eV, 330°C): m/e 684 ($M^+$)

669 ($M^+$-15)

158 (base peak)

A solution consisting of 4 g of the compound, 10 g of a thermoplastic polyester resin ("Vylon 200" produced by Toyobo Co., Ltd.) and 100 g of methyl ethyl ketone was coated on a 100 μm thick polyester film (produced by DIAFOIL Co., Ltd.) by means of a bar coater No. 3, followed by drying at 75°C for 10 minutes.

The resulting photosensitive material was colorless in an ordinary state but turned to deep blue ($\lambda_{max}$=610 nm) on irradiation of ultraviolet light. The absorption spectrum of the developed color is shown in Fig. 2. Then, it returned to its original colorless state on irradiation of visible light or heating at 80°C or higher. These color changes could be effected repeatedly.

EXAMPLE 10

In the same manner as in Example 9, except for using 9.4 g of dibromoethane in place of the dibromobutane, a compound of formula shown below was obtained.

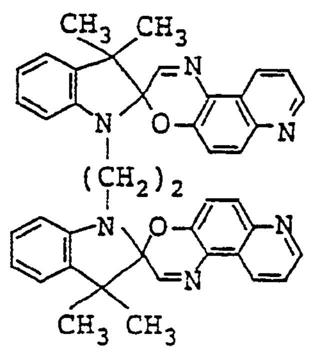

Mass Spectrum are as follows.

(Measurement Condition 70 eV, 300°C): m/e 656 ($M^+$)

641 ($M^+$-15)

158 (base peak)

A photosensitive material was produced by using the compound in the same manner as in Example 9. The sample developed a deep blue color ($\lambda_{max}$=610 nm) on ultraviolet irradiation and returned to its original colorless state on visible light irradiation or heating to 80°C or higher. These color changes could be effected repeatedly.

EXAMPLE 11

In 1000 ml of methyl ethyl ketone was dissolved 42.0 g of a compound of formula:

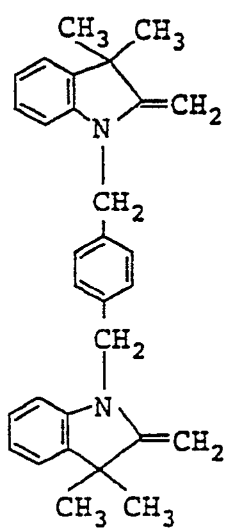

and to the solution was added 34.6 g of a compound of formula:

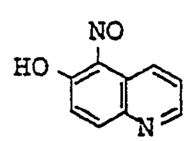

The mixture was refluxed for 3 hours and then cooled to room temperature. The solvent was removed by

distillation, and the residue was purified by column chromatography (benzene as an eluent) to obtain a spiro-oxazine compound of formula:

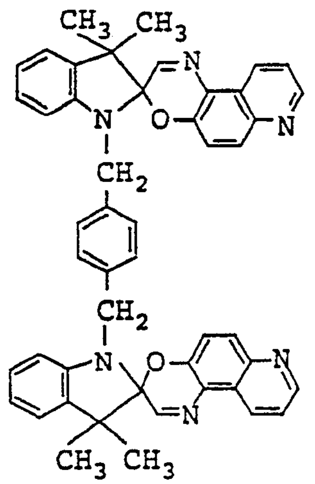

Mass Spectrum are as follows.

(Measurement Condition 70 eV, 350°C): m/e 732 ($M^+$)

717 ($M^+$-15)

158 (base peak)

A photosensitive material was produced by using the compound in the same manner as in Example 9. The resulting sample developed a deep blue color ($\lambda_{max}$=610 nm) on ultraviolet irradiation and then returned to its original colorless state on visible light irradiation or heating to 80°C or higher. These color changes could be effected repeatedly.

EXAMPLE 12

In the same manner as in Examples 9 to 11, spiro-oxazine compounds of formula (I) shown in Table 4 below were synthesized. The melting point and heat decomposition temperature of these compounds are also shown in Table 4.

A photosensitive material was produced by using each of these compounds in the same manner as in Example 9. Upon ultraviolet irradiation, the sample developed a color having tone and $\lambda_{max}$ as shown in Table 4 and, on visible light irradiation or heating to 80°C or higher it returned to its original colorless state. These color changes could be effected repeatedly.

TABLE 4

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-1 | 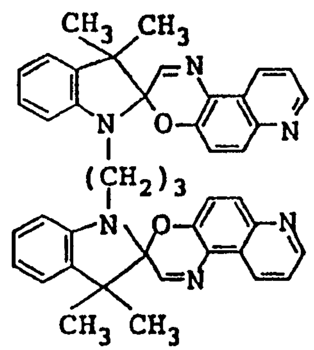 | Blue | 610 | | |
| 12-2 | 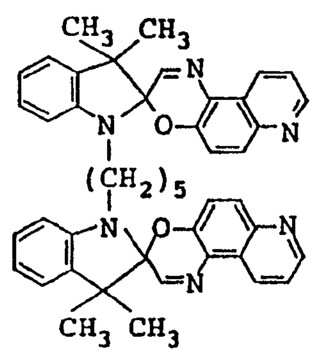 | Blue | 610 | | |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-3 | 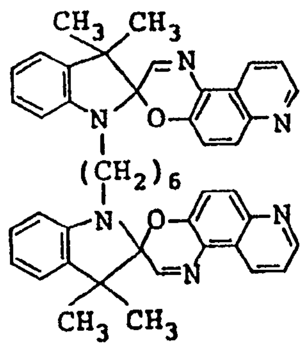 | Blue | 610 | 223 | 268 |
| 12-4 | 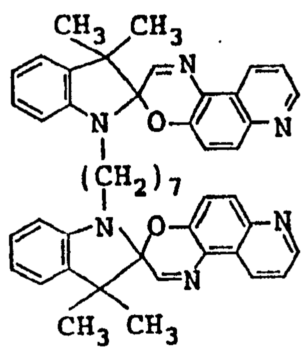 | Blue | 610 | 221 | 271 |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-5 | 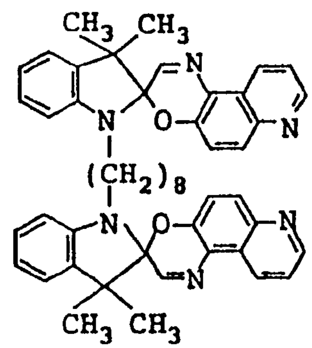 | Blue | 610 | 219 | 270 |
| 12-6 | 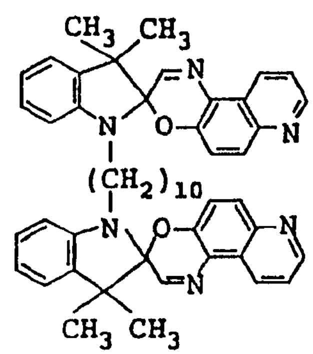 | Blue | 610 | 219 | 271 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-7 | 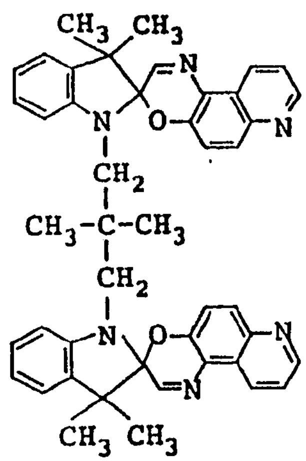 | Blue | 610 | 228 | 273 |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-8 | 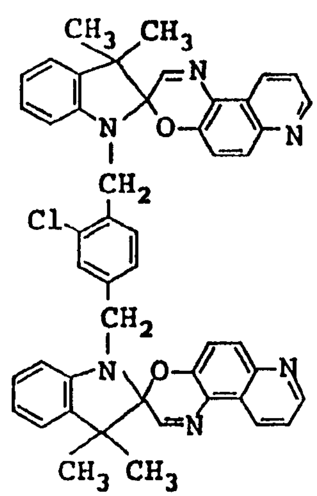 | Blue | 610 | 234 | 275 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-9 | 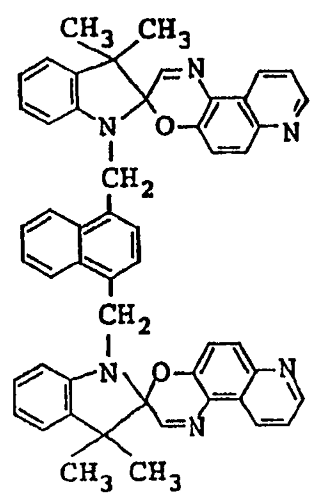 | Blue | 610 | 227 | 280 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-10 | 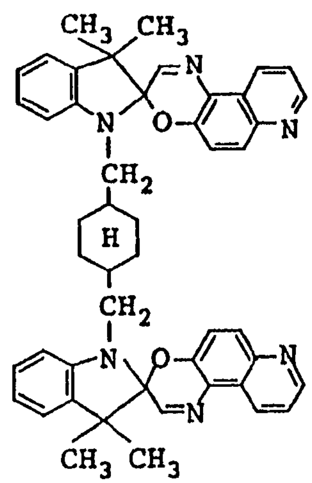 | Blue | 610 | 226 | 269 |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-11 | 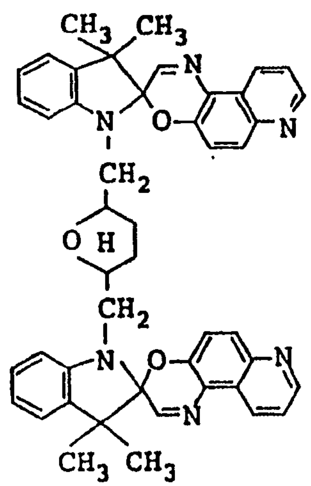 | Blue | 610 | 230 | 258 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-12 | 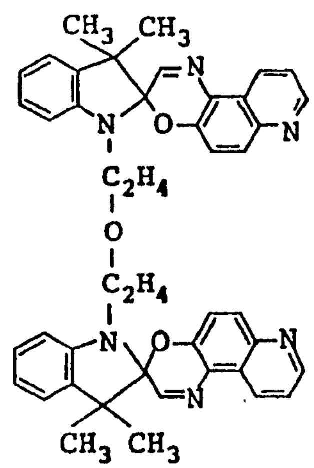 | Blue | 610 | 228 | 261 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-13 | 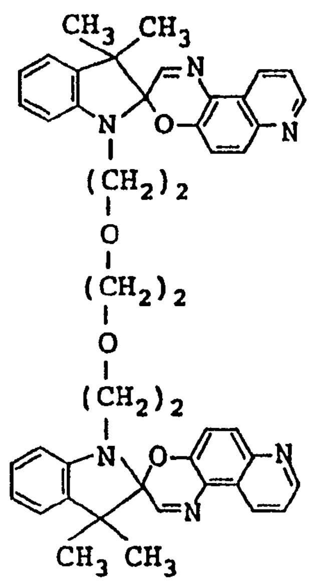 | Blue | 610 | 221 | 268 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-14 | 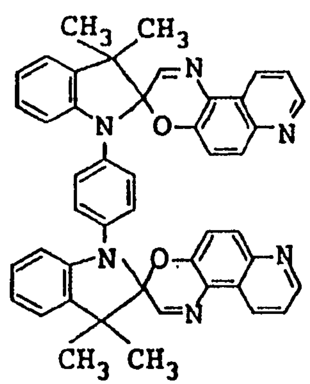 | Blue | 610 | 234 | 273 |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-15 | 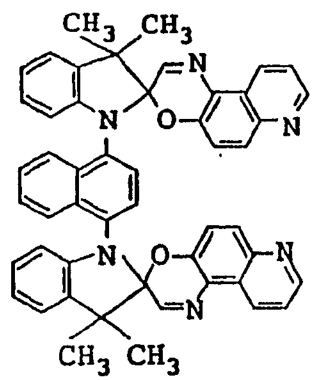 | Blue | 610 | 236 | 278 |
| 12-16 | 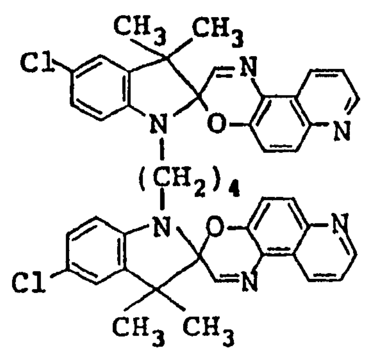 | Blue | 615 | 227 | 269 |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-17 | 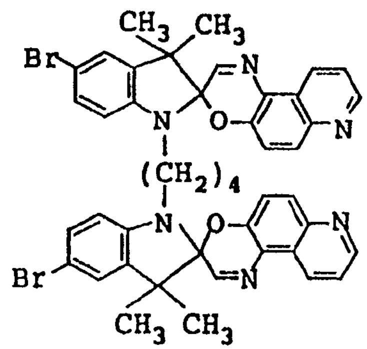 | Blue | 615 | 231 | 271 |
| 12-18 | 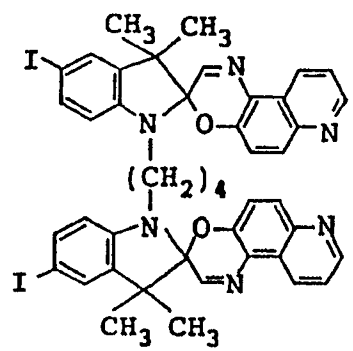 | Blue | 615 | 233 | 272 |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-19 | 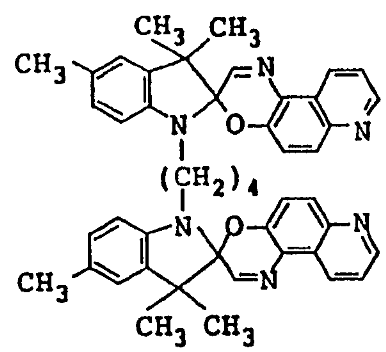 | Blue | 613 | 221 | 268 |
| 12-20 | 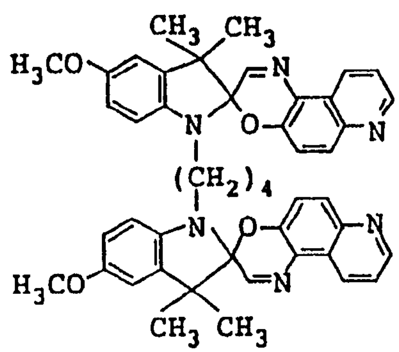 | Blue | 605 | 224 | 270 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-21 | 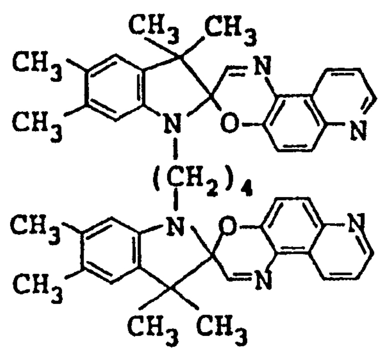 | Blue | 615 | 224 | 268 |
| 12-22 | 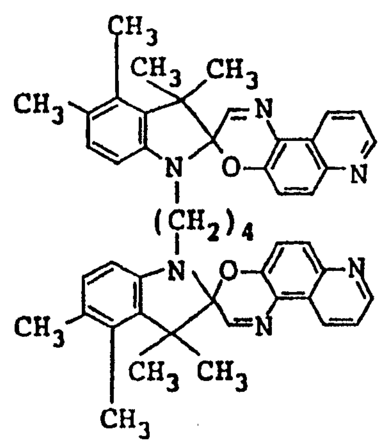 | Blue | 615 | 224 | 267 |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-23 | 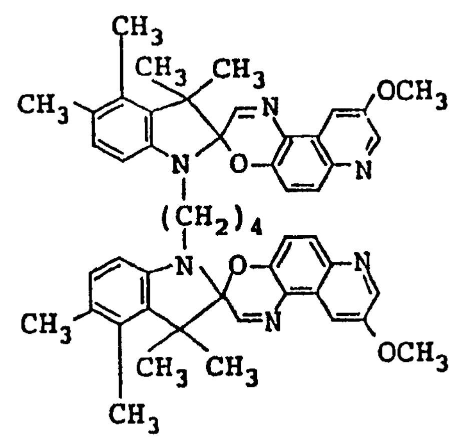 | Blue | 615 | | |
| 12-24 | 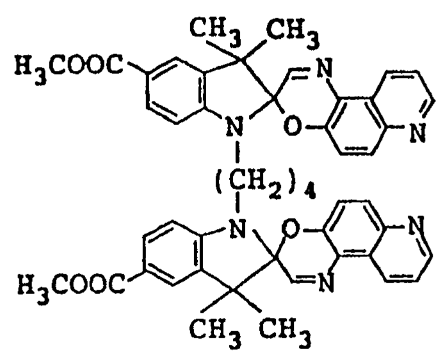 | Blue | 620 | | |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-25 | 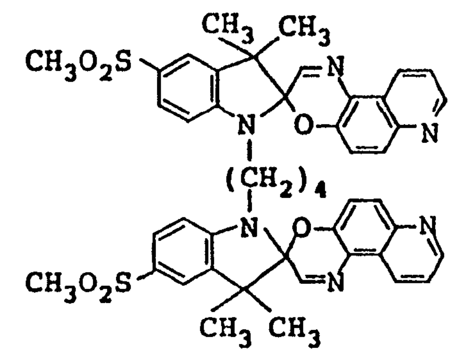 | Blue | 620 | | |
| 12-26 | 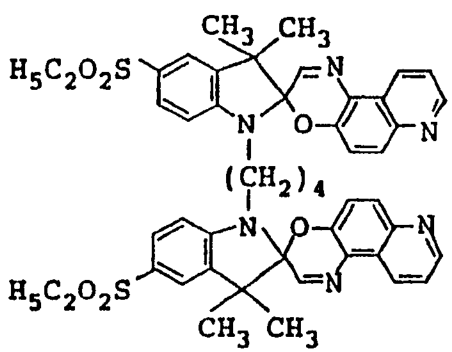 | Blue | 617 | | |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-27 | 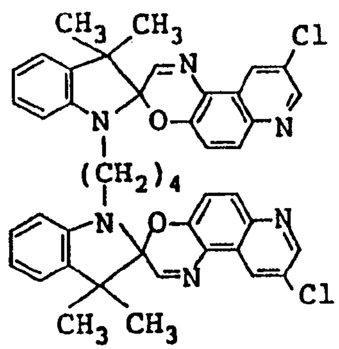 | Blue | 610 | | |
| 12-28 | 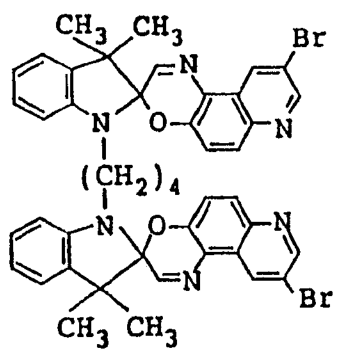 | Blue | 610 | | |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-29 | 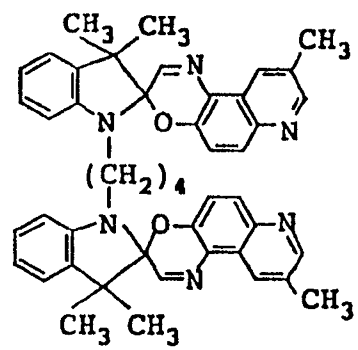 | Blue | 610 | | |
| 12-30 | 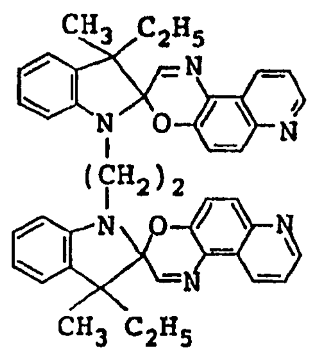 | Blue | 610 | | |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-31 | 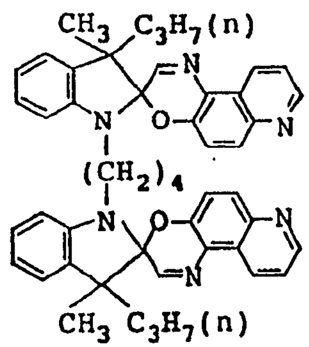 | Blue | 610 | | |
| 12-32 | 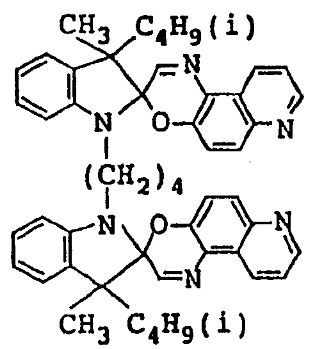 | Blue | 610 | | |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-33 | 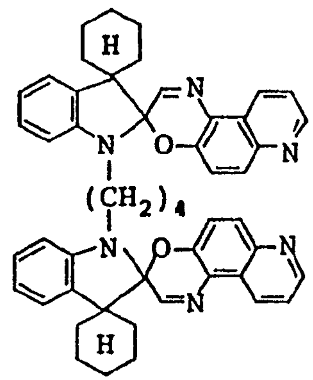 | Blue | 610 | | |
| 12-34 | 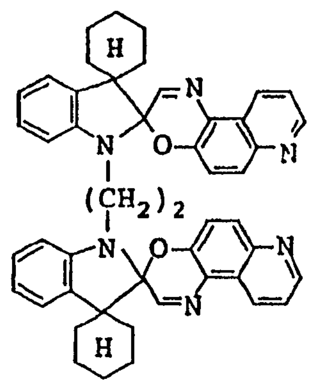 | Blue | 610 | | |

TABLE 4 (cont'd)

| Example No. | Spiro-Oxazine Compound Synthesized | Developed Color on Polyester Film by Irradiating UV Ray: Tone | Developed Color on Polyester Film by Irradiating UV Ray: λmax (nm) | Melting Point (°C) | Heat Decomposition Temperature (°C) |
|---|---|---|---|---|---|
| 12-35 | 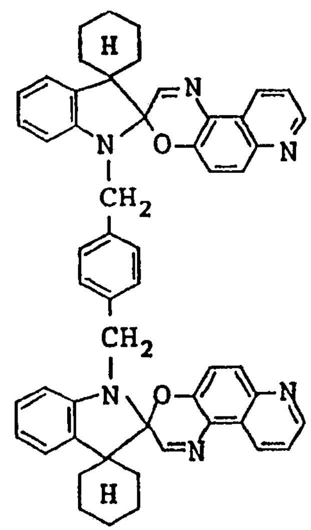 | Blue | 610 | | |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-36 | 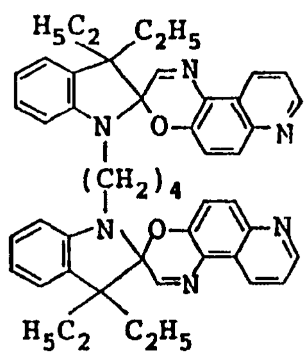 | Blue | 610 | | |
| 12-37 | 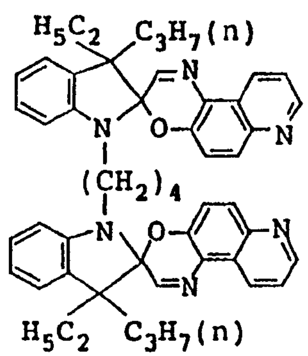 | Blue | 610 | | |

TABLE 4 (cont'd)

| Example No. | Spiro-Oxazine Compound Synthesized | Developed Color on Polyester Film by Irradiating UV Ray: Tone | λmax (nm) | Melting Point (°C) | Heat Decomposition Temperature (°C) |
|---|---|---|---|---|---|
| 12-38 | 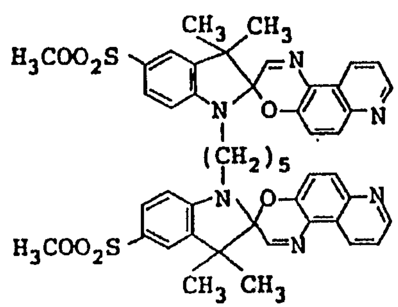 | Blue | 617 | | |
| 12-39 | 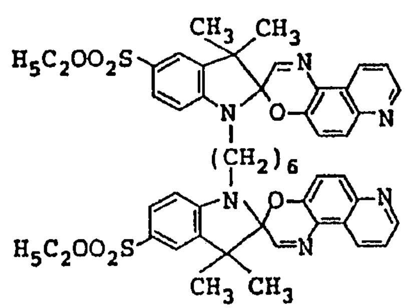 | Blue | 617 | | |

EXAMPLE 13

Ten grams of the photochromic compound of Example 4-1 shown in Table 2 (designated as SP-2) were added to 990 g of a polyvinylbutyral (PVB) powder, and 250 g of 3GH was added thereto. The mixture was meltmixed at 204°C and extruded from an extruder through a die to obtain a film having a thickness of 380 µm.

The resulting film was interposed between a pair of glass plates in a usual laminating method to produce a laminated glass for use in motor cars. For comparison, a laminated glass was produced in the same manner, except for using SP-1 in place of SP-2.

The laminated glass was irradiated with light emitted from a xenon lamp (30,000 lux) in a sunshine weatherometer, "UXL-75D" manufactured by Ushio Electric Ind. The change of optical density (ΔOD) at 610 nm with irradiation time is shown in Fig. 3. In Fig. 3, the ordinate indicates ΔOD, and the abscissa indicates an irradiation time (sec); A is light-on, and B is light-off.

SP-1 exhibits substantially no photochromism because it has been evaporated or decomposed during incorporation into the high-molecular material due to its low melting point and a low heat decomposition temperature.

Then, a mixture comprising 3 g of SP-2, 82 g of PVB, and 15 g of 3GH was melt-mixed in the same manner as described above. The molten mixture was extruded to obtain a film having a thickness of 760 µm (film①) or 380 µm (film②) or cast to obtain a film having a thickness of 50 µm (film③). Each of the film samples was irradiated in the same manner as described above, and ΔOD at 610 nm was determined. The results are shown in Table 5.

TABLE 5

| Sample No. | Molding Method | Film Thickness (µm) | ΔOD at 610 nm: Initial Stage of Irradiation | ΔOD at 610 nm: After 1000 Hrs' Irradiation |
|---|---|---|---|---|
| ① | extrusion | 760 | 0.31 | 0.27 |
| ② | extrusion | 380 | 0.25 | 0.18 |
| ③ | casting | 50 | 0.19 | 0.01 |

Weather resistance of photochromic compounds can generally be enhanced as the film thickness increases. In this connection, the results of Table 5 prove that the compound of the present invention and the film formation or molding process of the present invention are advantageous in favor of weather resistance as compared with the fact that the film thickness reached by the conventional film formation technique was about 100 µm at the most.

EXAMPLE 14

A mixture of 50 g of the compound having a quinoline ring as synthesized in Example 9 (designated as SP-3) and 950 g of a polymethyl methacrylate (PMMA) powder were melt-mixed at 210°C and pelletized in a pelletizer. The resulting pellets were injection-molded at 240°C to obtain a lens for glasses.

For comparison, a lens was obtained in the same manner, except for replacing SP-3 with a spiro-oxazine derivative having a quinoline ring as represented by formula:

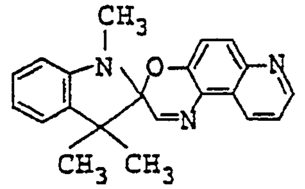

(designated as SP-4).

Each of the lenses was evaluated for ΔOD at 610 nm in the same manner as in Example 13, and the results obtained are shown in Fig. 4. In Fig. 4, A and B have the same meanings as in Fig. 3. It can be seen from Fig. 4 that SP-3 exhibits desired photochromism, whereas SP-4 exhibits no photochromism because it has been heat decomposed during pelletization or molding.

Industrially practical availability

As described above, the spiro-oxazine compounds according to the present invention exhibits photochromism and satisfactory heat resistance and develop a color having a high density and fastness. The photosensitive materials obtained by using the spiro-oxazine compounds of the invention are useful in various fields as light control materials, optical filters, masking materials, actinometers, etc.

Further, since the spiro-oxazine compounds of the invention have high melting points and satisfactory heat resistance, they can be processed not only by the conventional coating techniques but by various molding techniques after melt-mixing with a high-molecular matrix material at a high temperature. Application of the molding techniques makes it possible (a) to form a photochromic layer having a large thickness thereby exhibiting weather resistance, to increase a productivity thereby decreasing the cost, (b) to obtain a photochromic photosensitive material having a complicated shape, and (c) which could not be produced by the conventional coating techniques.

## Claims

1. A spiro-oxazine compound represented by formula (I):

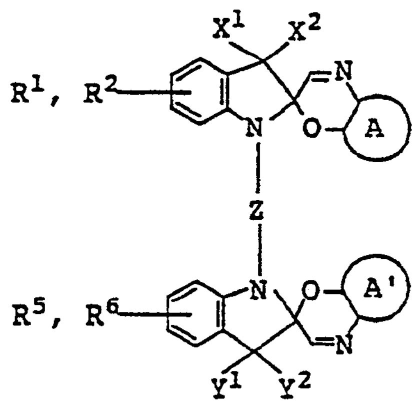

(I)

[wherein A and A′, which are the same, represent

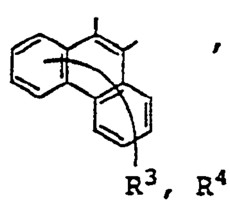

,

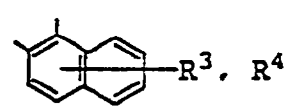

or

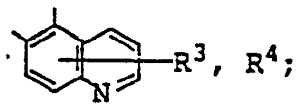

$X^1$, $X^2$ ,$Y^1$, and $Y^2$ each represents an alkyl group, or $X^1$ and $X^2$, or $Y^1$, and $Y^2$ are linked to each other to form a cyclohexane ring; Z represents a straight chain or branched chain alkylene group which may contain therein a group selected from

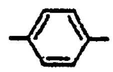 , 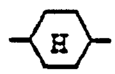 , 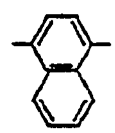

and

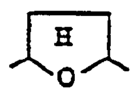 ,

each of which may have a substituent, or an oxygen atom, or a substituted or unsubstituted arylene group; and $R^1$, $R^2$, $R^3$, $R^4$, $R^5$, and $R^6$ each represents a hydrogen atom, a halogen atom, an alkyl group, an alkoxy group, an alkoxycarbonyl group, an alkoxysulfonyl group, or an alkylsulfonyl group].

2. A spiro-oxazine compound of the formula (I) as claimed in claim 1, wherein $R^1$, $R^2$, $R^5$, and $R^6$ each represents a hydrogen atom, a halogen atom, an alkyl group having from 1 to 6 carbon atoms, an alkoxycarbonyl group having from 1 to 6 carbon atoms, an alkylsulfonyl group having from 1 to 6 carbon atoms, or an alkoxysulfonyl group having from 1 to 6 carbon atoms; and $R^3$ and $R^4$ each represents a hydrogen atom, an alkoxy group having from 1 to 6 carbon atoms, a halogen atom, or an alkyl group having from 1 to 6 carbon atoms.

3. A spiro-oxazine compound of the formula (I) as claimed in claim 1, wherein $R^1$, $R^2$, $R^5$, and $R^6$ each represents a hydrogen atom, an alkyl group having from 1 to 4 carbon atoms, an alkoxycarbonyl group having from 1 to 4 carbon atoms, an alkylsulfonyl group having from 1 to 4 carbon atoms, or an alkoxysulfonyl group having from 1 to 4 carbon atoms; and $R^3$ and $R^4$ each represents a hydrogen atom, an alkoxy group having from 1 to 4 carbon atoms, a halogen atom, or an alkyl group having from 1 to 4 carbon atoms.

4. A spiro-oxazine compound of the formula (I) as claimed in claim 3, wherein $R^1$, $R^2$, $R^5$, and $R^6$ each represents a hydrogen atom, an alkyl group having 1 or 2 carbon atoms, an alkoxycarbonyl group having 1 or 2 carbon atoms, an alkylsulfonyl group having 1 or 2 carbon atoms, or an alkoxysulfonyl group having 1 or 2 carbon atoms; and $R^3$ and $R^4$ each represents a hydrogen atom, an alkoxy group having 1 or 2 carbon atoms, a halogen atom, or an alkyl group having 1 or 2 carbon atoms.

5. A spiro-oxazine compound of the formula (I) as claimed in claim 1, wherein $X^1$, $X^2$, $Y^1$, and $Y^2$ each represents an alkyl group having from 1 to 4 carbon atoms, or $X^1$ and $X^2$, or $Y^1$ and $Y^2$ are linked to each other to form a cyclohexane ring.

6. A spiro-oxazine compound of the formula (I) as claimed in claim 1, wherein Z represents $-(CH_2)_n-$, $-CH_2-Q-CH_2-$, or

$$-[(CH_2)_mO]_l-(CH_2)_m-,$$

wherein Q represents

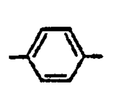 , 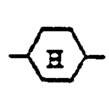 ,

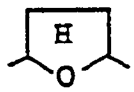

or

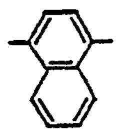

n represents an integer of from 1 to 10; and ℓ and m each represents 1 or 2.

7. A spiro-oxazine compound of the formula (I) as claimed in claim 2, wherein $X^1$, $X^2$, $Y^1$, and $Y^2$ each represents a methyl group; and Z represents $-(CH_2)_{n''}-$, wherein n is from 4 to 8.

8. A spiro-oxazine compound of the formula (I) as claimed in claim 7, wherein A and A', which are the same, represent

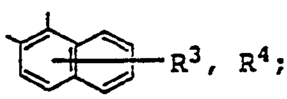

$R^1$, $R^2$, $R^5$, and $R^6$ each represents a hydrogen atom or a methyl group; and $R^3$ and $R^4$ each represents a hydrogen atom or a methoxy group.

9. A photosensitive material comprising a base having provided thereon a photosensitive layer containing a resin having dissolved or dispersed therein a spiro-oxazine compound of the formula (I) as claimed in claim 1.

10. A photosensitive material as claimed in claim 9, wherein the spiro-oxazine compound of the formula (I) is as claimed in claim 7.

11. A photosensitive material as claimed in claim 9, wherein the spiro-oxazine compound of the formula (I) is as claimed in claim 8.

12. A process for producing a photosensitive material comprising incorporating a spiro-oxazine compound of the formula (I) as claimed in claim 1 into a high-molecular material at a high temperature, followed by molding thereof.

13. A process as claimed in claim 12, wherein the spiro-oxazine compound of the formula (I) is as claimed in claim 7.

14. A process as claimed in claim 12, wherein in formula (I) A and A', which are the same, represent

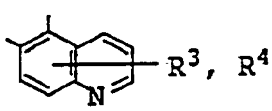

or A, A', $R^3$, $R^4$, $R^1$, $R^2$, $R^5$, and $R^6$ are as defined in claim 8.

15. A process as claimed in claim (12), wherein said high-molecular material is an acrylic polymer or polyvinyl butyral.

## Patentansprüche

1. Spiro-Oxazin-Verbindung der Formel (I)

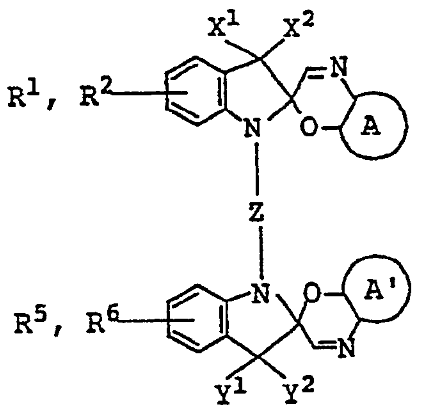

(I)

(in der A und A', welche gleich sind, die Reste

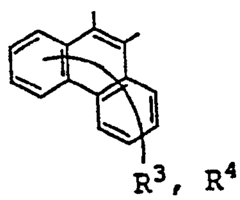

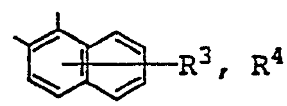

oder

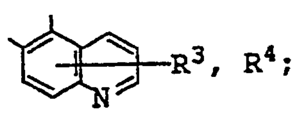

bedeuten, $X^1$, $X^2$, $Y^1$ und $Y^2$ jeweils Alkylgruppen bedeuten, oder $X^1$ und $X^2$ oder $Y^1$ und $Y^2$ miteinander zu einem Cyclohexanring verbunden sind, Z einen linearen oder verzweigten Alkylenrest darstellt, der eine Gruppe gewählt aus

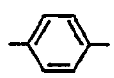 , 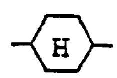 , 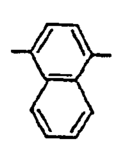

und



enthalten kann, wovon jede einen Substituenten oder ein Sauerstoffatom oder einen substituierten oder unsubstituierten Arylenrest tragen kann, und $R^1$, $R^2$, $R^3$, $R^4$, $R^5$ und $R^6$ jeweils ein Wasserstoffatom, ein Halogenatom, einen Alkylrest, einen Alkoxyrest, einen Alkoxycarbonylrest, einen Alkoxysulfonylrest oder einen Alkylsulfonylrest darstellen).

2. Spiro-Oxazin-Verbindung der Formel (I) nach Anspruch 1, in der $R^1$, $R^2$, $R^5$ und $R^6$ jeweils ein Wasserstoffatom, ein Halogenatom, einen Alkylrest mit 1 bis 6 Kohlenstoffatomen, einen Alkoxycarbonylrest mit 1 bis 6 Kohlenstoffatomen, einen Alkylsulfonylrest mit 1 bis 6 Kohlenstoffatomen oder einen Alkoxysulfonylrest mit 1 bis 6 Kohlenstoffatomen darstellen und $R^3$ und $R^4$ jeweils ein Wasserstoffatom, einen Alkoxyrest mit 1 bis 6 Kohlenstoffatomen, ein Halogenatom oder eine Alkylgruppe von 1 bis 6 Kohlenstoffatomen darstellen.

3. Spiro-Oxazin-Verbindung der Formel (I) nach Anspruch 1, in der $R^1$, $R^2$, $R^5$ und $R^6$ jeweils ein Wasserstoffatom, einen Alkylrest mit 1 bis 4 Kohlenstoffatomen, einen Alkoxycarbonylrest mit 1 bis 4 Kohlenstoffatomen, einen Alkylsulfonylrest mit 1 bis 4 Kohlenstoffatomen oder einen Alkoxysulfonylrest mit 1 bis 4 Kohlenstoffatomen darstellen und $R^3$ und $R^4$ jeweils ein Wasserstoffatom, einen Alkoxyrest mit 1 bis 4 Kohlenstoffatomen, ein Halogenatom oder einen Alkylrest von 1 bis 4 Kohlenstoffatomen darstellen.

4. Spiro-Oxazin-Verbindung der Formel (I) nach Anspruch 3, in der $R^1$, $R^2$, $R^5$ und $R^6$ jeweils ein Wasserstoffatom, einen Alkylrest mit 1 oder 2 Kohlenstoffatomen, einen Alkoxycarbonylrest mit 1 oder 2 Kohlenstoffatomen, einen Alkylsulfonylrest mit 1 oder 2 Kohlenstoffatomen oder einen Alkoxysulfonylrest mit 1 oder 2 Kohlenstoffatomen darstellen und $R^3$ und $R^4$ jeweils ein Wasserstoffatom, einen Alkoxyrest mit 1 oder 2 Kohlenstoffatomen, ein Halogenatom oder einen Alkylrest mit 1 oder 2 Kohlenstoffatomen darstellen.

5. Spiro-Oxazin-Verbindung der Formel (I) nach Anspruch 1, in der $X^1$, $X^2$, $Y^1$ und $Y^2$ jeweils einen Alkylrest mit 1 bis 4 Kohlenstoffatomen darstellen oder $X^1$ und $X^2$ oder $Y^1$ und $Y^2$ sind miteinander zu einem Cyclohexanring verbunden sind.

6. Spiro-Oxazin-Verbindung der Formel (I) nach Anspruch 1, in der Z $-(CH_2)_n-$, $-CH_2-Q-CH_2-$ oder $-[(CH_2)_mO]_\ell-(CH_2)_m-$, bedeutet, wobei Q

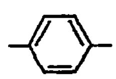 , 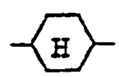 , 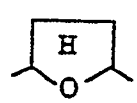

oder

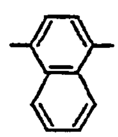

ist, bedeutet n eine ganze Zahl von 1 bis 10 und $\ell$ und m jeweils 1 oder 2 bedeuten.

7. Spiro-Oxazin-Verbindung der Formel (I) nach Anspruch 2, in der $X^1$, $X^2$, $Y^1$ und $Y^2$ jeweils eine Methylgruppe bedeuten, und Z $-(CH_2)_n-$ bedeutet, wobei n die Zahl 4 bis 8 darstellt.

8. Spiro-Oxazin-Verbindung der Formel (I) nach Anspruch 7, in der A und A', welche gleich sind, den Rest

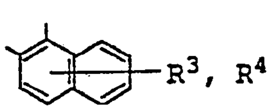

darstellen, $R^1$, $R^2$, $R^5$ und $R^6$ jeweils ein Wasserstoffatom oder eine Methylgruppe darstellen, und $R^3$ und $R^4$ jeweils ein Wasserstoffatom oder eine Methoxygruppe darstellen.

9. Photoempfindliches Material, umfassend einen Träger und darauf eine photoempfindliche Schicht, enthaltend ein Harz, in dem eine Spiro-Oxazin-Verbindung der Formel (I) nach Anspruch 1 gelöst oder dispergiert enthalten ist.

10. Photoempfindliches Material nach Anspruch 9, in dem die Spiro-Oxazin-Verbindung der Formel (I) gemäß Anspruch 7 vorliegt.

11. Photoempfindliches Material nach Anspruch 9, in dem die Spiro-Oxazin-Verbindung der Formel (I) gemäß Anspruch 8 vorliegt.

12. Verfahren zur Herstellung eines photoempfindlichen Materials, anfassend die Einverleibung einer Spiro-Oxazin-Verbindung der Formel (I) nach Anspruch 1 in ein hochmolekulares Material bei hoher Temperatur und anschließendes Formen desselben.

13. Verfahren nach Anspruch 12, in dem die Spiro-Oxazin-Verbindung der Formel (I) gemäß Anspruch 7 vorliegt.

14. Verfahren nach Anspruch 12, wobei in der Formel (I) A und A', welche gleich sind, den Rest

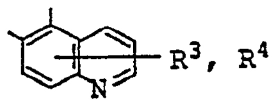

bedeuten, oder A, A', $R^3$, $R^4$, $R^1$, $R^2$, $R^5$ und $R^6$ wie in Anspruch 8 definiert sind.

15. Verfahren nach Anspruch 12, wobei das hochmolekulare Material ein Acryl-Polymer oder ein Polyvinylbutyral ist.

## Revendications

1. Composé de spiro-oxazine représenté par la formule (I) :

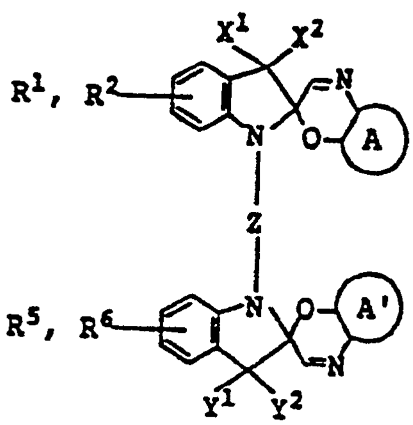

(I)

[dans laquelle : A et A', qui sont identiques, représentent

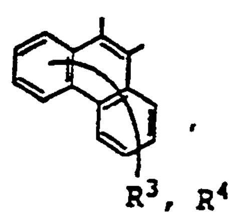

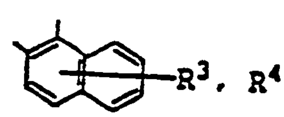

ou

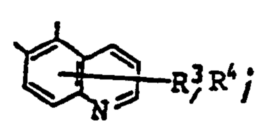

$X^1$, $X^2$, $Y^1$ et $Y^2$ représentent chacun un groupe alkyle, ou bien $X^1$ et $X^2$, ou $Y^1$ et $Y^2$, sont reliés l'un à l'autre

pour former un noyau cyclohexane ; Z représente un groupe alkylène à chaîne droite ou à chaîne ramifiée, qui peut contenir en son sein un groupe choisi parmi

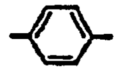 , 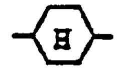 , 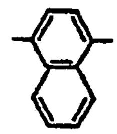

et

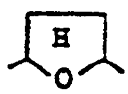 ,

chacun pouvant avoir un substituant, ou un atome d'oxygène, ou un groupe arylène substitué ou non substitué ; et $R^1$, $R^2$, $R^3$, $R^4$, $R^5$ et $R^6$ représentent chacun un atome d'hydrogène, un atome d'halogène, un groupe alkyle, un groupe alcoxy, un groupe alcoxycarbonyle, un groupe alcoxysulfonyle ou un groupe alkylsulfonyle].

2. Composé de spiro-oxazine de la formule (I), selon la revendication 1, dans lequel : $R^1$, $R^2$, $R^5$ et $R^6$ représentent chacun un atome d'hydrogène, un atome d'halogène, un groupe alkyl ayant de 1 à 6 atomes de carbone, un groupe alcoxycarbonyle ayant de 1 à 6 atomes de carbone, un groupe alkylsulfonyle ayant de 1 à 6 atomes de carbone, ou un groupe alcoxysulfonyle ayant de 1 à 6 atomes de carbone ; et $R^3$ et $R^4$ représentent chacun un atome d'hydrogène, un groupe alcoxy ayant de 1 à 6 atomes de carbone, un atome d'halogène, ou un groupe alkyle ayant de 1 à 6 atomes de carbone.

3.Composé de spiro-oxazine de la formule (I), selon la revendication 1, dans lequel : $R^1$, $R^2$, $R^5$ et $R^6$ représentent chacun un atome d'hydrogène, un groupe alkyle ayant de 1 à 4 atomes de carbone, un groupe alcoxycarbonyle ayant de 1 à 4 atomes de carbone, un groupe alkylsulfonyle ayant de 1 à 4 atomes de carbone, ou un groupe alcoxysulfonyle ayant de 1 à 4 atomes de carbone ; et $R^3$ et $R^4$ représentent chacun un atome d'hydrogène, un groupe alcoxy ayant de 1 à 4 atomes de carbone, un atome d'halogène, ou un groupe alkyle ayant de 1 à 4 atomes de carbone.

4. Composé de spiro-oxazine de la formule (I), selon la revendication 3, dans lequel : $R^1$, $R^2$, $R^5$ et $R^6$ représentent chacun un atome d'hydrogène, un groupe alkyl ayant 1 ou 2 atomes de carbone, un groupe alcoxycarbonyle ayant 1 ou 2 atomes de carbone, un groupe alkylsulfonyle ayant 1 ou 2 atomes de carbone, ou un groupe alcoxysulfonyle ayant 1 ou 2 atomes de carbone ; et $R^3$ et $R^4$ représentent chacun un atome d'hydrogène, un groupe alcoxy ayant 1 ou 2 atomes de carbone, un atome d'halogène, ou un groupe alkyle ayant 1 ou 2 atomes de carbone.

5. Composé de spiro-oxazine de la formule (I), selon la revendication 1, dans lequel $X^1$, $X^2$, $Y^1$ et $Y^2$ représentent chacun un groupe alkyle ayant de 1 à 4 atomes de carbone, ou bien $X^1$ et $X^2$, ou $Y^1$ et $Y^2$, sont reliés l'un à l'autre pour former un noyau cyclohexane.

6. Composé de spiro-oxazine de la formule (I), selon la revendication 1, dans lequel Z représente $-(CH_2)n$, $-CH_2-Q-CH_2-$, ou

$$-[(CH_2)_mO]_\ell-(CH_2)_m,$$

où : Q représente

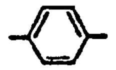 , 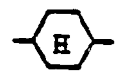 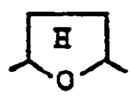

ou

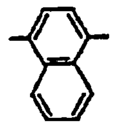

;

n représente un nombre entier allant de 1 à 10 ; et ℓ et m représentent chacun 1 ou 2.

7. Composé de spiro-oxazine de la formule (I), selon la revendication 2, dans lequel : $X^1$, $X^2$, $Y^1$ et $Y^2$ représentent chacun un groupe méthyle ; et Z représente $-(CH_2)_n-$, où n va de 4 à 8.

8. Composé de spiro-oxazine de la formule (I), selon la revendication 7, dans lequel : A et A', qui sont identiques , représentent

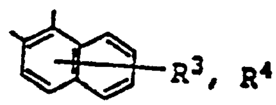

;

$R^1$, $R^2$, $R^5$ et $R^6$ représentent chacun un atome d'hydrogène ou un groupe méthyle ; et $R^3$ et $R^4$ représentent chacun un atome d'hydrogène ou un groupe méthoxy.

9. Matière photosensible comprenant une base sur laquelle est disposée une couche photosensible contenant une résine dans laquelle est dissous ou dispersé un composé de spiro-oxazine de la formule (I) selon la revendication 1.

10. Matière photosensible selon la revendication 9, dans laquelle le composé de spiro-oxazine de la formule (I) est tel que défini à la revendication 7.

11. Matière photosensible selon la revendication 9, dans laquelle le composé de spiro-oxazine de la formule (I) est tel que défini à la revendication 8.

12. Procédé de fabrication d'une matière photosensible comprenant l'incorporation d'un composé de spiro-oxazine de la formule (I) selon la revendication 1, dans une matière de masse moléculaire élevée, à température élevée, suivie du moulage de celle-ci.

13. Procédé selon la revendication 12, dans lequel le composé de spiro-oxazine de la formule (I) est tel que défini à la revendication 7.

14. Procédé selon la revendication 12, dans lequel, dans la formule (I), A et A' qui sont identiques, représentent

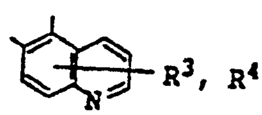

ou A, A', $R^3$, $R^4$, $R^1$, $R^2$, $R^5$ et $R^6$ sont tels que définis à la revendication 8.

15. Procédé selon la revendication 12, dans lequel ladite matière de masse moléculaire élevée est un polymère acrylique ou du polyvinyl butyral.

Fig. 1

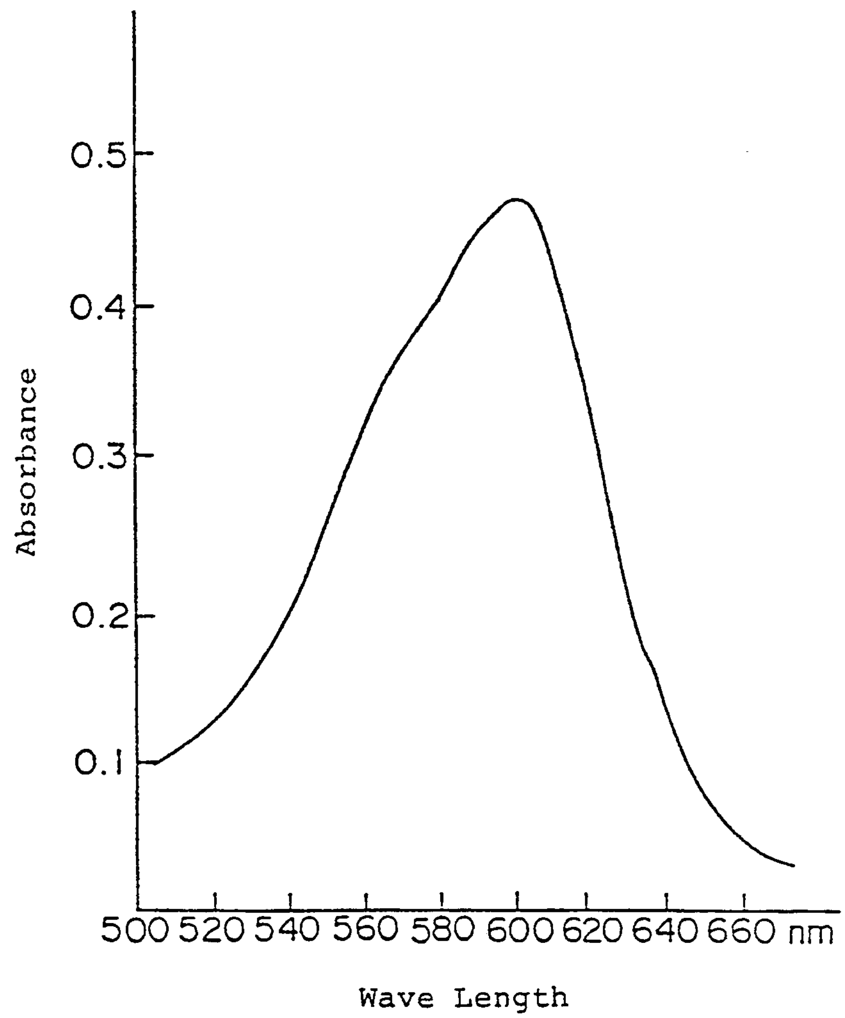
0.5
0.4
0.3
0.2
0.1
Absorbance
500 520 540 560 580 600 620 640 660 nm
Wave Length

Fig. 2

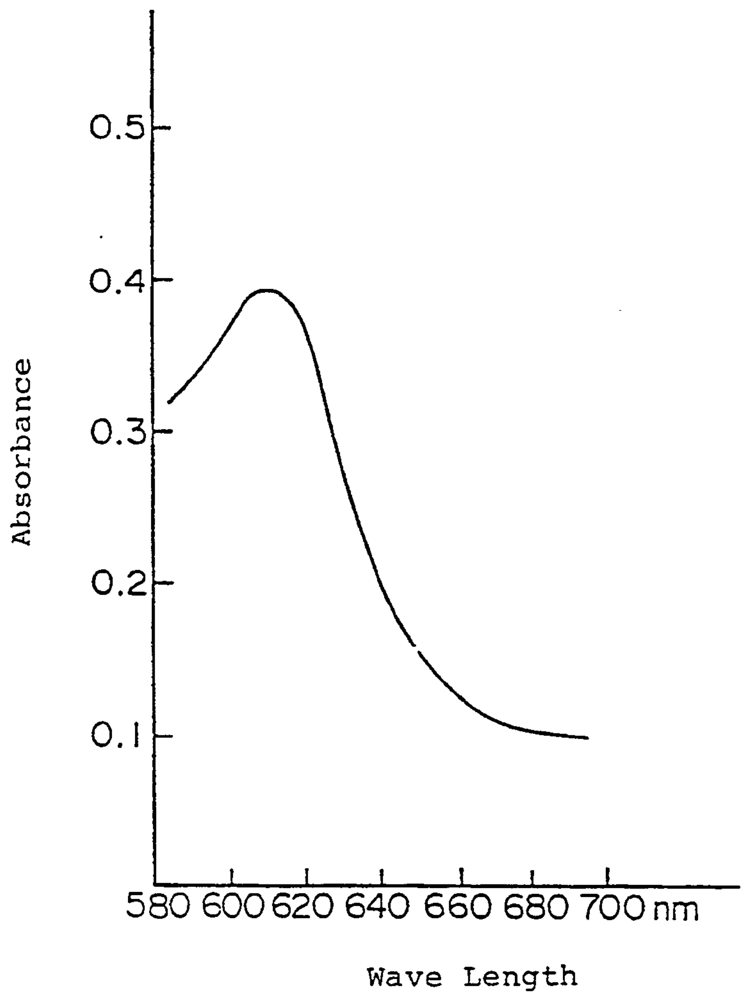
0.5
0.4
0.3
0.2
0.1
Absorbance
580 600 620 640 660 680 700 nm
Wave Length

Fig. 3

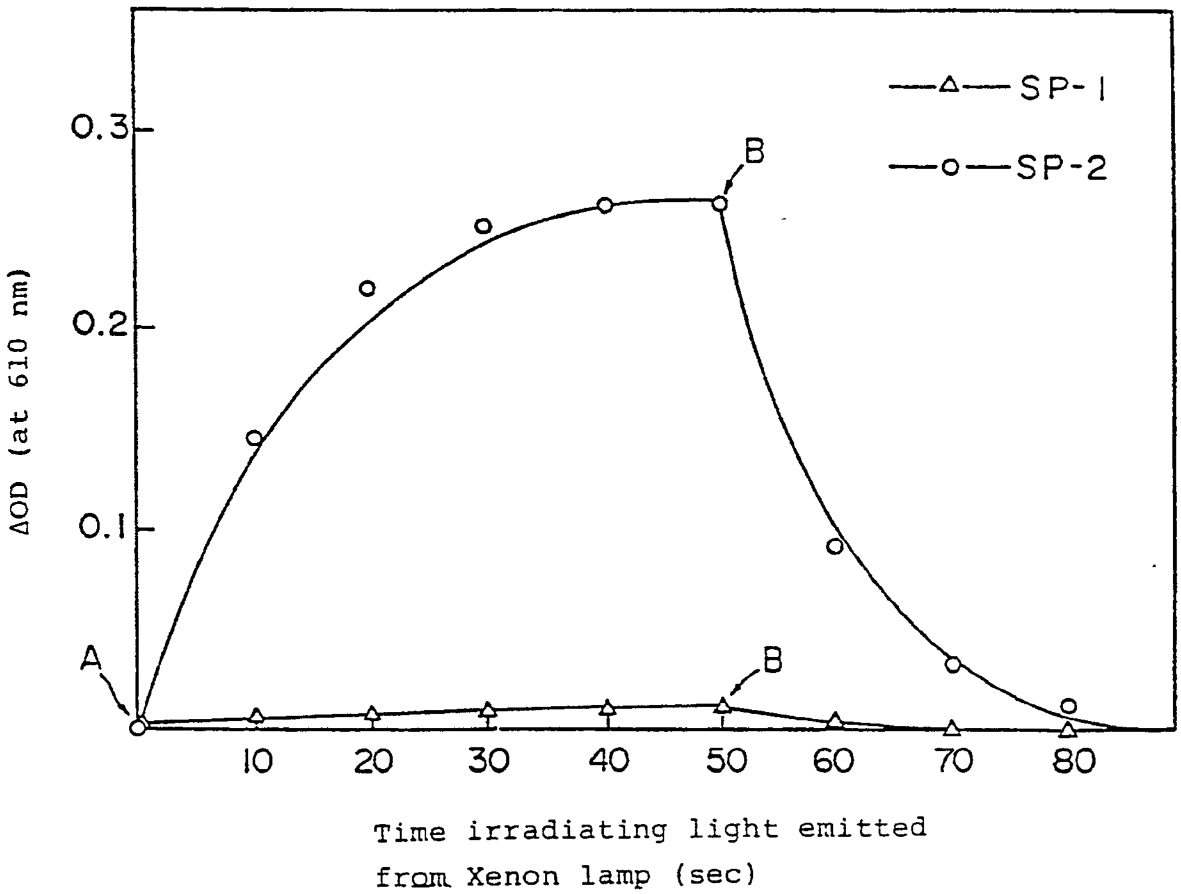

SP-1
SP-2
0.3
0.2
0.1
A
B
B
ΔOD (at 610 nm)
10
20
30
40
50
60
70
80
Time irradiating light emitted
from Xenon lamp (sec)

Fig. 4

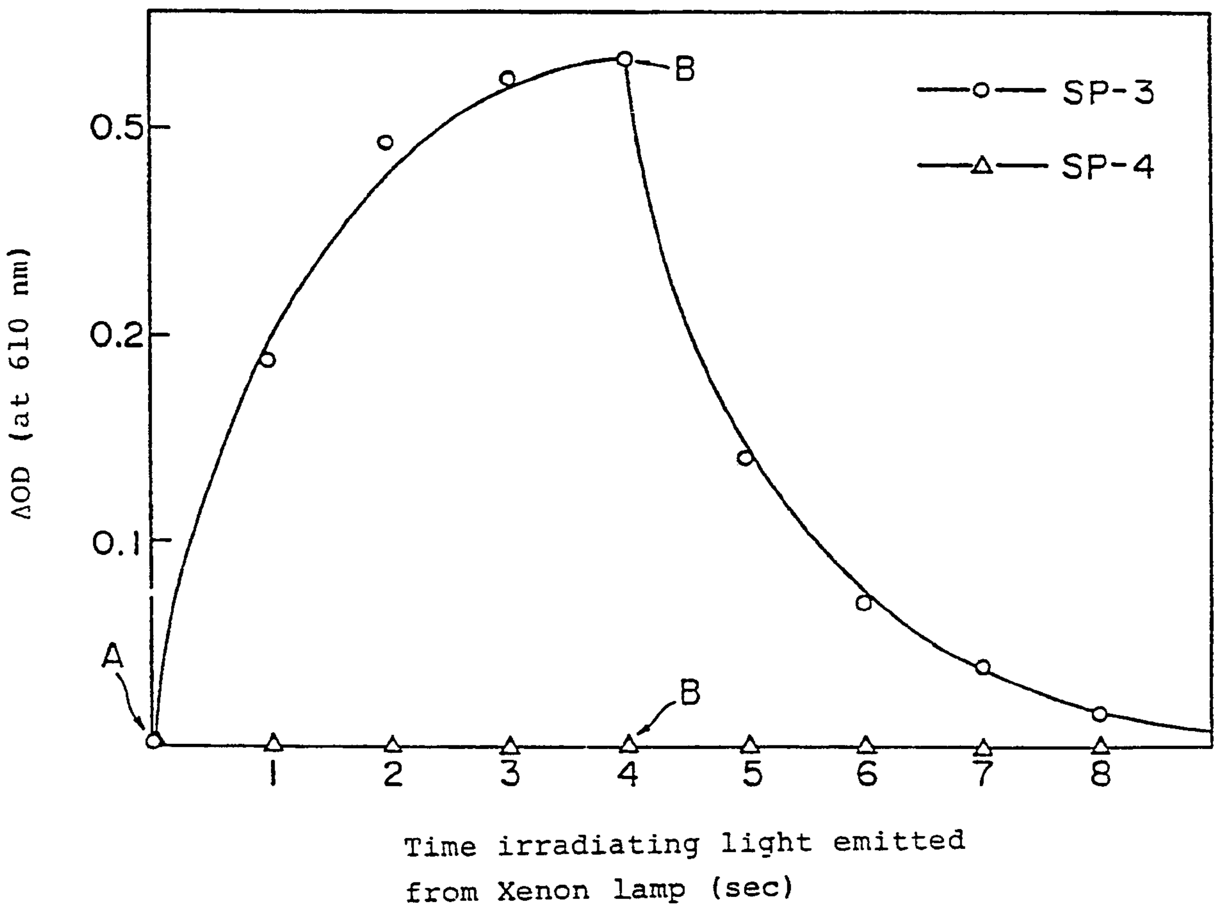
ΔOD (at 610 nm)
0.5
0.2
0.1
A
B
B
—o— SP-3
—Δ— SP-4
1
2
3
4
5
6
7
8
Time irradiating light emitted from Xenon lamp (sec)